# EUROPEAN PATENT APPLICATION

(11) **EP 4 847 079 A1**
(43) Date of publication of application: **02.12.2026**
(21) Application number: 26465525.9
(22) Date of filing: 19.03.2026
(51) Int. Cl.: A61N 1/04, A61M 37/00, A61N 1/30, B82Y 30/00, A61N 1/32

(54) **MICRONEEDLE-BASED SYSTEM FOR TRANSDERMAL LOADING AND DELIVERY OF THERAPEUTIC AGENTS**

(71) Applicant: Universitatea Nationala de Stiinta si Tehnologie Politehnica Bucuresti, Bucharest (RO)
(72) Inventor: GHITMAN, Jana, 061126 Bucharest (RO); COBIANU, Cornel, 051434 Bucharest (RO); ILIESCU, Florina Silvia, 077180 Tunari, Ilfov (RO); IOVU, Horia, 050121 Bucharest (RO); ILIESCU, Ciprian, 077180 Tunari, Ilfov (RO)
(74) Representative: Fierascu, Cosmina-Catrinel

(57) **Abstract**

The invention relates to an iontophoresis-enhanced transdermal delivery system integrating a flexible, conductive, and biocompatible microneedle (MN) array with a biodegradable nanofibrous reservoir containing positively charged nanoparticle-encapsulated therapeutic agents. The system comprises an anodic MN patch and a cathodic MN patch including a flexible and electrically conductive base layer having a plurality of microneedles disposed thereon, both formed from a carbon-reinforced crosslinked polymeric material selected from polyimides (PI), polyamides (PA), or polydimethylsiloxane (PDMS). The incorporation of conductive fillers from the carbon family, such as carbon black, reduced graphene oxide, carbon nanotubes, carbon nanohorns, carbon nano onions, or carbon fibers, at concentrations above the percolation threshold provides high electrical conductivity and mechanical reinforcement. The anodic MN patch is coated with a crosslinked, biodegradable, and biocompatible electrospun active nanofibrous layer forming the reservoir while the cathodic MN patch is reservoir-free. This reservoir comprises a polymeric matrix based on methacrylated gelatin (GelMA), in which positively charged nanoparticles loaded with therapeutic agents are embedded and/or surface deposited. The nanoparticles may include polyplexes, lipoplexes, or drug-loaded cationic nanocarriers, designed to stabilize and protect the therapeutic cargo and facilitate iontophoretic transdermal delivery. The invention further discloses methods for fabricating and assembling the MN system. The disclosed fabrication process is cost-effective, scalable, and suitable for mass production, offering improved bioavailability, enhanced pharmacological efficacy, and increased patient compliance.

## Description

### 1. TECHNICAL FIELD

The present disclosure generally relates to devices for the iontophoresis-enhanced transdermal delivery of drugs and genetic materials, including genes and nucleic acids. In particular, the invention relates to a flexible, electrically conductive and water-insoluble microneedle array incorporating a biodegradable and biocompatible fibrous reservoir, enabling high loading capacity and efficient transdermal delivery of nano-formulated therapeutic agents with enhanced therapeutic efficacy. The present disclosure also relates to a method for the efficient transdermal delivery of therapeutic agents using an electrically- conductive microneedle array comprising a biodegradable and biocompatible fibrous reservoir in combination with iontophoresis.

### 2. BACKGROUND OF THE INVENTION

Transdermal delivery of therapeutic agents using microneedles (MN) represents a useful method for achieving pharmacological and therapeutic effects both systemically and locally. Transdermal administration of therapeutic agents offers several advantages over traditional delivery methods, such as oral or subcutaneous administration, by avoiding first-pass metabolism and reducing patient discomfort associated with subcutaneous injections. Also, transdermal administration can provide uniform concentrations of therapeutic agents in the bloodstream by modulating the delivery profile of the transdermal device used. Microneedles are notable for enabling non-invasive or minimally invasive delivery of therapeutic agents through the skin. The advantage of using microneedles lies in the fact that the delivery of therapeutic agents can be achieved without pain, discomfort and improving patient compliance. Microneedles are able to penetrate the stratum corneum barrier of the skin to create micropores, thus allowing the easy permeation of therapeutic agent molecules. A wide variety of microneedles, including silicon and metal microneedles, coated microneedles, and biodegradable, water-soluble, or water-insoluble microneedles, have been tested depending on the application and the desired therapeutic effect. However, reduced therapeutic efficacy and challenges in achieving rapid and controlled delivery rates of therapeutics agents, which are necessary to ensure increased concentrations at the target site, remain significant limitations in the field.

Various methods have been investigated in microneedle-based devices for transdermal applications to increase the concentration and delivery rate of therapeutic agents, including the use of chemical permeation enhancers and iontophoresis. Transdermal iontophoresis is a non-invasive technique that uses a voltage source to generate a low electrical current and a repulsive electromotive force, facilitating the transport of ionized drugs or therapeutic agents across the skin. The combination of microneedle technology with iontophoresis represents a potent approach for delivering increased concentrations of various therapeutic agents through the skin in a controlled manner. In such systems, therapeutic agents can be incorporated within or around the microneedle matrix, and upon application of an electrical current, the agents are driven through the micropores created by the microneedles and into the skin, enabling enhanced permeation and controlled delivery.

Vemulapalli et al. (Vemulapalli, Viswatej, Ye Yang, Phillip M. Friden, and Ajay K. Banga. "Synergistic effect of iontophoresis and soluble microneedles for transdermal delivery of methotrexate. "Journal of Pharmacy and Pharmacology" 60, no. 1 (2008): 27-33; 10.1211/jpp.60.1.0004 - 2008 - 004) demonstrated a 25-fold increase in the in vivo delivery of methotrexate by employing a synergistic combination of maltose dissolvable microneedles with iontophoresis (0.4 mA/cm² for 60 minutes) compared to either method used alone.

In another study (Chen, Huabing, Hongda Zhu, Jingnan Zheng, Dongsheng Mou, Jiangling Wan, Junyong Zhang, Tielin Shi, Yingjun Zhao, Huibi Xu, and Xiangliang Yang. "Iontophoresis-driven penetration of nanovesicles through microneedle-induced skin microchannels for enhancing transdermal delivery of insulin. "Journal of controlled release"139, no. 1 (2009): 63-72; doi.org/10.1016/j.jconrel.2009.05.031), microneedle-induced skin microchannels combined with iontophoresis were used to enhance the delivery efficiency of cationic nanovesicle-encapsulated insulin. Microneedles were applied prior to iontophoresis to create microchannels, facilitating delivery pathways, while iontophoresis subsequently drove the charged nanovesicles through the microchannels. This combination demonstrated a synergistic effect, achieving approximately a 713-fold increase in insulin flux compared to passive diffusion alone and enabling rapid and effective delivery sufficient to reduce blood glucose levels in streptozotocin-induced diabetic rats.

Arshad et al. (Arshad, Muhammad Sohail, Saad Hussain, Saman Zafar, Sadia Jafar Rana, Nadia Ahmad, Najmusama Abdul Jalil, and Zeeshan Ahmad. "Improved transdermal delivery of rabies vaccine using iontophoresis coupled microneedle approach. "Pharmaceutical Research" 40, no. 8 (2023): 2039-2049; doi.org/10.1007/s11095-023-03521-0) combined solid microneedle pretreatment with iontophoresis to improve the transdermal delivery and immunogenicity of a rabies vaccine as a potential needle-free, patient-friendly vaccination method. The patches, fabricated from polyvinylpyrrolidone, hyaluronic acid, and polyethylene glycol, showed strong mechanical stability and effective skin penetration. In vitro, the microneedle-iontophoresis system nearly doubled drug distribution compared to microneedles alone. *In vivo,* rats showed controlled epidermal penetration without tissue damage, while dogs demonstrated significantly stronger immune responses. Microneedles alone increased IgG titers by about 73%, whereas the combined system boosted them by more than 200%, with neutralizing antibody levels also markedly higher. The study concluded that microneedle vaccination coupled with iontophoresis provides a powerful, noninvasive alternative to conventional intramuscular rabies immunization.

Hu et al. (Hu, Tianli, Zhibiao Zhang, and Chenjie Xu. "Transdermal delivery of dextran using conductive microneedles assisted by iontophoresis. "Journal of Materials Chemistry B" 10, no. 39 (2022): 8075-8081; doi.org/10.1039/D2TB01049F) reported improved delivery of macromolecular dextrans in agarose gel utilizing a conductive microneedle array fabricated from polyaniline and hyaluronic acid in combination with iontophoresis.

Su et al. (Su, Yajuan, Valerio Luca Mainardi, Hongjun Wang, Alec McCarthy, Yu Shrike Zhang, Shixuan Chen, Johnson V. John et al. "Dissolvable microneedles coupled with nanofiber dressings eradicate biofilms via effectively delivering a database-designed antimicrobial peptide. "ACS Nano" 14, no. 9 (2020): 11775-11786; DOI: 10.1021/acsnano.0c04527) developed a dual-component delivery platform, referred to as a Janus-type antimicrobial dressing, comprising W379 peptide-incorporated dissolvable microneedle arrays immobilized on the surface of peptide-loaded electrospun nanofiber membranes. The system featured a specialized design in which electrospun antimicrobial peptide-loaded polycaprolactone (PCL) nanofibers were deposited directly onto biocompatible and dissolvable polyvinylpyrrolidone (PVP) microneedles. This configuration enhanced antimicrobial peptide delivery and biofilm penetration, enabling effective targeting of both the interior and exterior regions of biofilms present in chronic wounds, while significantly reducing bacterial counts in vivo diabetic wound models. In this work the authors used dissolvable microneedles coupled with nanofiber dressings to deliver a database-designed antimicrobial peptide directly into biofilms. The nanofibrous dressing serves as a supportive and carrier element, providing structural integration with the microneedles and a passive platform for holding and releasing the antimicrobial peptide, where release occurs solely via microneedle dissolution and peptide diffusion from the nanofiber, without any iontophoresis-mediated modulation. Although the system offers enhanced diffusion of the peptide, it does not allow precise temporal or dosage control and therefore cannot ensure optimal bioavailability of the encapsulated agents.

A recent study (Wang, Ting, Xiaoyu Shi, Yue Wang, Hengda Sun, Yu Sun, Gang Wang, and Hong Jiang. "Lidocaine-loaded iontophoresis-driven fiber-based microneedle patch for controllable and long-lasting transdermal local analgesia. "Advanced Fiber Materials" 7, no. 1 (2025): 281-295; doi.org/10.1007/s42765-024-00486-6) reported the development of a smart, controllable microneedle patch integrating dissolvable microneedles composed of electrospun cotton fibers, iontophoresis, and a smartphone-controlled system to achieve precise, adjustable, and sustained delivery of lidocaine for local analgesia. The dissolvable microneedles were fabricated using pure cotton fiber with gel layers on the tips for high-capacity loading of lidocaine at the microneedle tips and surfaces. Enhanced transdermal delivery of lidocaine was achieved through a combination of passive diffusion *via* microneedle-created microchannels and active transport *via* iontophoresis (<0.5 mA/cm²). The system enabled user-defined control of delivery profiles-including single-dose, pulsed, or sustained release-through a smartphone application, allowing for on-demand, precisely timed analgesic administration. The synergistic use of microneedles and iontophoresis significantly improved drug flux and extended the duration of analgesic effect compared to either approach alone. The platform demonstrated strong potential for personalized, non-invasive pain management in postoperative, dental, and dermatological applications requiring controllable and sustained local analgesia. It should be noted that, although lidocaine delivery in this system is achieved through a combination of passive diffusion and iontophoresis, the microneedles themselves are not conductive, being fabricated from a dielectric polymeric dissolvable cotton fiber canvas, while the conductive pathway required for establishing the iontophoretic circuit is provided exclusively by the PVA-PEDOT:PSS hydrogel layer integrated on (only) the surface of the patch. Therefore, there is no continuous conductive path from outer electrodes to the tips of the needles. Such an approach with dielectric microneedles may limit the efficiency of the iontophoresis process, due to ultralow electric currents through the iontophoresis circuit on the skin. Furthermore, while the authors addressed electrical safety and skin irritation under varying current conditions, the long-term safety of this approach may need more extensive evaluation.

EP3681584B1discloses a dissolving microneedle array for transdermal drug delivery (TDD) of macromolecules (APIs) using iontophoresis. The microneedles (MN) are made of water-soluble polymers (poly(methyl vinyl ether-maleic acid)), humectants (glycerol), and surfactants (polysorbate 80), encapsulating APIs within them. Upon application to the skin, microneedles dissolve, and iontophoresis is applied using low electrical current to enhance delivery rate and reduce lag time of encapsulated drugs.
Surprisingly, the authors of this patent are using iontophoresis method for drug delivery, but the base and the microneedles of the transdermal patch are made of pure dielectric materials (electrically isolating materials), which are preventing the electric current to flow through the closed electric circuit composed of power source-external anode- patch with MN- substratum corneum- cathode-power source. The efficiency of the iontophoresis for their process is almost zero, due to ultralow the electric current (tens of picoamperes) flowing through the above dc biased electric circuit.

US007763203B2 describes micromolds made from photoresist materials or PDMS (polydimethylsiloxane) enable rapid, low-cost production of polymer microneedle arrays with various designs (solid, hollow, detachable, electrode-embedded) via molding or casting, used in transdermal delivery, sampling, and biosensing with minimal pain. This patent protects the micromolding technology based on PDMS as a negative scaffold for the MN fabrication.

US20210106520A1/US11607383B2 discloses a method for delivering therapeutic agents using a microneedle array having a conductive coating that includes a therapeutic agent and a conducting polymer, such as polypyrrole (PPy) or PEDOT. The system employs electrical stimulation (iontophoresis) to control the release of the therapeutic agent across the dura mater for delivery to the central nervous system. The microneedles are fabricated from biocompatible polymers and are initially gold-coated to create a metallic seed layer which will further allow the electrodeposition of the conducting polymer layer containing the therapeutic agent (e.g., dexamethasone) onto the microneedle surface. The above technological process is indeed adequately designed for the efficient application of the iontophoresis to the MN-based TDD device, as it allows the electric current to flow from the power source through electrically conductive layers (metal, e.g. gold, and conductive polymers incorporating the drugs) covering the patch and the MN's, which can be even isolating materials (example needles made of polymeric IPS material). However, their technology is not fully reliable, as the gold cannot be directly deposited on polymeric needles! In addition, their whole technology is complex and very expensive, containing separate gold layer sputtering processes for covering both the microneedles surface, the vertical edges of the substrate and the opposite face of the substrate, as well as multiple electrodeposition processes for the preparation of the conductive polymeric coating containing also the drug components.

WO2020060495A1 discloses a comprehensive transdermal drug delivery system that combines microneedles with iontophoresis to actively enhance and control the delivery of therapeutic agents, including macromolecules, through the skin with minimal invasiveness and improved bioavailability. The polymeric conductive microneedles consist of swellable and water-insoluble matrix or water-soluble matrix comprising a crosslinked polymer and incorporating also a conducting polymer such as polypyrrole, PEDOT:PSS, and polyaniline enabling them to function as electrodes during iontophoresis. The therapeutic agents can be applied to the skin following microneedle insertion, embedded within dissolvable microneedles for direct release, or incorporated into formulations containing permeation enhancers. Controlled, on-demand delivery rates is claimed via electrophoresis and/or electroosmosis under the applied electrical field. Although for a working iontophoresis enhanced drug delivery system, it is essential that the base of the MN patch to be also electrically conductive, so that a continuous conducting path from the power source to the tip of the microneedle to be obtained, it appears that the WO2020060495A1 invention does not consider this aspect in detail, and describes this aspect of conductive base only tangentially and in finally, only optionally for their patented MN patch. As will be shown in the next sections of our disclosed invention, the requirement for an electrically conductive base of the MN patch is equally important as the fabrication process for the conductive MN's.

Recently, a scientific paper (Samuel Mugo, Scott Robertson, and Weihao Lu, "Transdermal microneedle patch for antioxidants release", Discover Materials (2025) 5:64; doi.org/10.1007/s43939-025-00240-8) has described a flexible microneedle patch for iontophoresis (I2P) enhanced transdermal drug delivery where the base of the MN patch was made from a pure PDMS thick layer, while the conductive microneedles were made from cellulose nanocrystal doped with and polyaniline and carbon nano-tubes (CNT). For the I2P enhanced MN patch operation the authors must apply a DC voltage on the back surface of the PDMS layer and close the circuit through the second electrode. However, as the PDMS layer is an electrically isolating material, the I2P resulting electric current is practically equal to zero. Such a conclusion can be observed from their impedance spectroscopy results, where the resistive component of the impedance is quite high, and this is because of the contribution of the non-conducting PDMS!

As shown above, even if it is fully recognized that the transdermal drug delivery (TDD) via iontophoresis-enhanced MN patches is able to increase the efficiency of painless therapeutic agents transfer through the skin with respect to passive diffusion method, in addition enabling the delivery of therapeutic agents of much higher molecular masses, "there is plenty of room at the bottom" of the iontophoresis for the technology improvement and innovation in the field of TDD. The state of the art from above has shown either expensive technologies, or incomplete, even erroneous fabrication steps for an appropriate iontophoresis enhanced MN patch. In the same time, considering the therapeutic agent reservoir, there is a need for an improved protection of the sensitive biological molecules, such as nucleic acids or gene-based therapeutics, from degradation within the physiological environment, during formulation and deposition, facilitating a larger amount of agent and an easier integration technology of it into the large-scale fabrication of the iontophoresis enhanced MN-based transdermal delivery. Furthermore, although certain systems incorporate electronic control elements, they often depend on surface-applied drug formulations or lack integration with high-capacity and protective therapeutic agent reservoirs.

There is therefore a need for an improved, low cost, iontophoresis-enhanced MN-based transdermal delivery system with a simple and straightforward fabrication process on both MN patch and active nanofibrous reservoir, that overcomes one or more of the limitations and deficiencies associated with existing microneedle-iontophoresis technologies. The present invention addresses these deficiencies and provides additional advantages as described herein.

### 3. SUMMARY

The present invention relates to a low cost, novel iontophoresis-enhanced transdermal delivery system that integrates a flexible, conductive, biocompatible, water-insoluble substrate and microneedle (MN) array with a biodegradable and biocompatible nanofibrous reservoir containing positively charged nanoparticle-encapsulating therapeutic agents. The original fabrication method of such a iontophoresis-enhanced MN system will be also disclosed here, in terms of the (i) fabrication of the anodic MN patch defined here as consisting of electrically conductive and flexible substrate and microneedle disposed on its surface, (ii) novel technology for the active nanofibrous reservoir composition and fabrication, (iii) fabrication of the cathodic MN patch with the same technology, but without any therapeutic reservoir, and, (iv) reliable assembly technology of the MN patch-reservoir tandem on the skin, aiming safe and enhanced therapeutic agent delivery in the presence of an electric voltage source connected to the MN system, followed by the electrical connection of the anodic and cathodic MN patches, to the anode (positive terminal) and cathode (negative terminal) electrodes, respectively, of the voltage source. Such a straightforward, low-cost technology for both MN patches and reservoir fabrication can be easily up scalable to mass production. The use of biodegradable positively charged nanoparticles within the biodegradable fibrous reservoir stabilizes and protects the encapsulated therapeutic agents from premature degradation in biological environments, and facilitates efficient loading and controllable transdermal delivery via (i) electrostatic repulsion of the therapeutic agent-loaded positively charged nanoparticles towards the skin and (ii) electroosmosis flow processes thereby increasing the bioavailability and improving the pharmacological and therapeutic efficacy of the delivered therapeutic agents. Additionally, the system allows for dual-controlled, on-demand release kinetics via the synergistic combination of iontophoresis and reservoir matrix degradation, allowing for precise temporal and dosage regulation, thereby enhancing therapeutic efficacy and improving patient compliance.

In one embodiment, it is disclosed a flexible, fully electrically conductive MN patch (substrate and microneedle array) conceived for efficient iontophoresis enhanced transdermal drug delivery comprising:
- a flexible and electrically conductive base/substrate having a plurality of electrically conductive microneedles disposed thereon, wherein the base and the plurality of microneedles are formed from the same type of crosslinked polymer selected from polyimides, polyamides, or polydimethylsiloxane (PDMS), which are reinforced with carbonic nanomaterials for increasing their electrical and mechanical properties.
- a conductive filler/material from the carbon family, such as carbon black (CB), reduced graphene oxide (rGO), carbon nanotubes (CNT), carbon nanohorns (CNH), carbon fiber (CF) or carbon nano onion (CNO) incorporated at a concentration higher than the percolation threshold into the matrix of both the flexible substrate and the plurality of microneedles.

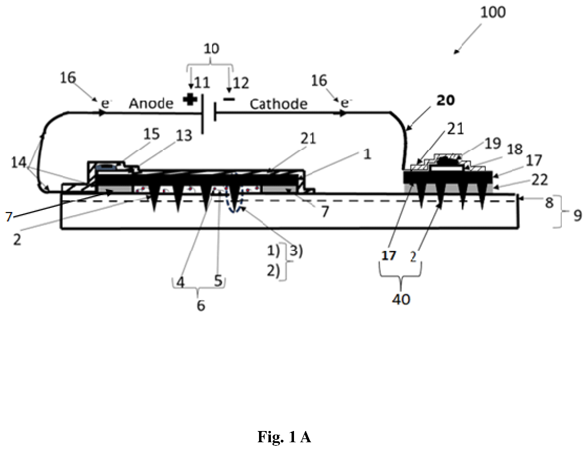

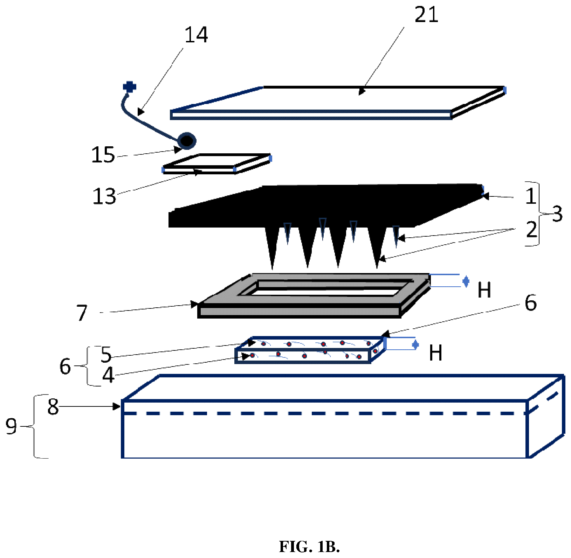

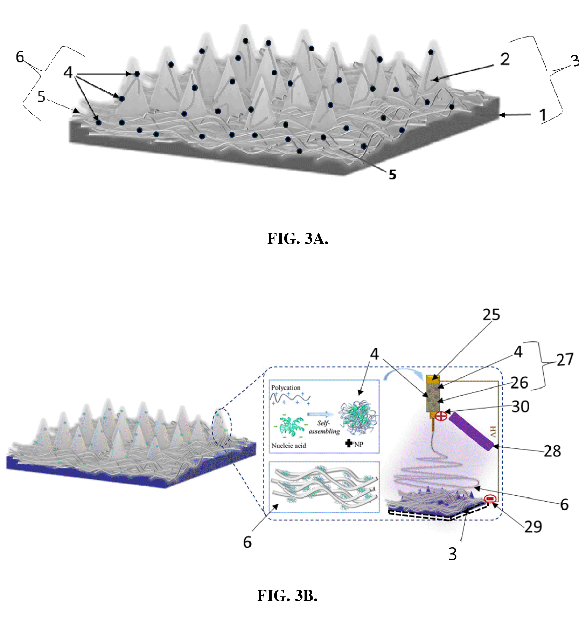

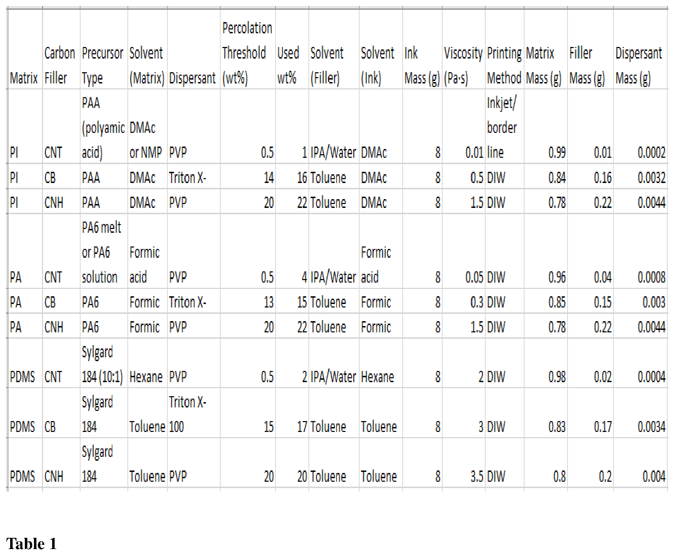

In other embodiments, there are disclosed different types of active nanofibrous reservoir, each of them prepared as a crosslinked, biodegradable electrospun nanofibrous polymeric layer consisting of:
- a naturally derived and chemically modified biocompatible and biodegradable polymeric-based matrix (e.g. methacrylated gelatine - GelMA);
- positively charged nanoparticles encapsulating the therapeutic agent, which are loaded inside and on the surface of the biodegradable electrospun nanofibrous polymeric layer, described above.
   These positively charged nanoparticles carrying therapeutic agents can be either (i) polyplexes formed by the interaction of a biocompatible polycationic nucleic acid carrier with a nucleic acid, or (ii) lipoplexes - obtained by assembling nucleic acids with biocompatible polycationic lipid carriers, or (iii) drug-loaded positively charged nanoparticles, obtained by assembling drugs with polycationic carriers.
   The therapeutic agents-loaded positively charged nanoparticles are embedded in the nanofibrous biodegradable and biocompatible reservoir, named herein active nanofibrous reservoir 6, (Fig. 1) which is deposited as a layer by electrospinning (i) either directly onto the surface of the MN substrate 1 and of the plurality of conductive microneedles 2 (Fig. 1), or, (ii) on the negatively-biased metallic collector 29 (Fig. 3B) of the electrospinning instrument followed by the subsequent layer transfer on the skin 9.

In another embodiment, it is disclosed a MN system 100 configured for the iontophoresis-enhanced transdermal delivery of one or more therapeutic agents, the MN system comprising:
- An electrically conductive and flexible MN patch, 3 (Fig. 1) (conductive base/substrate 1 having a plurality of conductive microneedles 2 disposed of thereon, wherein the base and the plurality of microneedles are formed from a carbon-reinforced crosslinked polymer selected from polyimides (PI), polyamides (PA), or polydimethylsiloxane (PDMS). The reinforcing of the substrate 1 and microneedles of the MN patch is made by incorporating conductive filler/ material from the carbon family, such as carbon black, reduced graphene oxide (rGO), carbon nanotubes (CNT), carbon nanohorns (CNH), carbon nano onions (CNO), carbon fibers (CB) into the matrix of both the flexible substrate 1 and the plurality of microneedles 2, at carbon concentrations higher than the percolation threshold, so that multiple conduction paths are formed for rendering high electrical conductivity and improved mechanical strength to both the substrate and microneedle array;
- Ag electrodes 13 deposited on the outer surface of the anodic MN patch 3 for being connected to positive terminal 11 of the voltage supply 10.
- Adhesive dielectric layer 7 positioned as a ring on the edge of the MN patch 3 for fixing the MN patch to the skin and sealing the therapeutic agent 6 inside the MN patch 3. The thickness of the adhesive layer 7 will be designed in correlation with the thickness of the anodic MN patch 3 and reservoir layer 6, so that a planar surface of the tandem MN patch-reservoir to be obtained after the insertion of the microneedle in the skin.
- A similar electrically conductive and flexible patch 40 (cathodic MN patch), provided with microneedles 2, but without any therapeutic reservoir layer, placed in the proximity of the anodic MN patch, and which is sealed to the skin 9 with an electrically conductive adhesive 22 (Fig. 1). This cathodic MN patch 40 will be provided with AgCl electrodes 18 deposited on the upper surface, opposite the surface containing microneedles 2 and used for being connected to the negative terminal 12 of the voltage source 10.

- An active nanofibrous reservoir 6 as defined above to be deposited either (i) directly onto the surface of the anodic MN patch 3 containing the conductive substrate 1 and the plurality of conductive microneedles 2 or (ii) on the negatively biased metallic collector 29 of the electrospinning instrument (Fig. 3B), followed by its transfer to the skin.
- A kinesiology tape 21 for sealing both the anodic and cathodic MN patches, and thus safely keeping a stable position of the microneedles after their insertion in the skin.
- An iontophoresis unit comprising a voltage source 10 with positive terminal 11 (anode) connectable to the anodic MN patch and its negative terminal 12 (cathode) to the cathodic MN patch 3, wherein the iontophoresis unit is configured to control an electrical current through the skin and drive the enhanced delivery of the therapeutic agents from the active nanofibrous reservoir 6 through the microneedle-created microchannels into the skin 9.

Another embodiment provides a method for transdermal delivery of one or more therapeutic agents using the transdermal delivery system 100 described herein, and the method consists of:
- applying and fixing with dielectric adhesive 7 on the surface of the skin the conductive anodic MN patch 3 coated with the active nanofibrous reservoir 6, the reservoir comprising positively charged nanoparticles 4 encapsulating the therapeutic agent; applying and fixing with conductive adhesive 22 the cathodic MN patch 40 (no reservoir layer present at this location) provided with AgCl electrode 18, 19, 20 (to be described later) to the skin 9, in the proximity of the anodic MN patch 3;
- connecting the electrodes of the anodic 3 and cathodic 40 MN patches to the positive11 and negative 12 terminals, respectively, of a voltage source 10 to establish an electrical circuit and allow a controllable electric current of about 0.5 mA to flow through the circuit containing the local dermal zone;
- activating an iontophoresis unit for operation at the optimal dc current so that to facilitate safe dual-controlled release of the therapeutic agent, wherein such dual-controlled delivery is governed both by (i) electrical stimulation, and (ii) the gradual degradation of the biodegradable active nanofibrous reservoir.

Another embodiment provides a method for transdermal delivery of one or more therapeutic agents using the iontophoresis-enhanced transdermal delivery device described herein, and the method comprises:
- separate fabrication followed by transfer and application of the preformulated biodegradable active nanofibrous reservoir 6 comprising fibers 5 and positively charged nanoparticles 4 loaded with the therapeutic agent, to the surface of the skin 9;
- applying and fixing the dielectric adhesive 7 to the surrounding (edge) area of the conductive MN patch 3 (already provided with electrode at the upper surface, i.e., opposite to the microneedles), so that the MN patch 9 to seal the reservoir inside its edges, while the dielectric adhesive will drive electric current flow only in the region of the microneedles;
- applying and fixing the electrically conductive adhesive 7 to the entire area of the cathodic MN patch 40 (provided with electrodes at the upper surface, i.e., opposite to the adhesive surface) to be placed in the proximity of the MN patch 3;
- connecting the electrode 13, 14, 15 (to be describe later) of the anodic MN patch 3 to positive terminal 11 of the voltage source 10 and the electrode of the cathodic MN patch 40 to the negative terminal 11 of the dc voltage source 10 so that to close the electric circuit and to establish a well-controlled electric current through the skin; and
- activating an iontophoresis unit to facilitate dual-controlled release of the therapeutic agent, wherein delivery is governed both by electrical stimulation at a controlled dc electric current of about 0.5 mA and by the gradual degradation of the biodegradable active nanofibrous reservoir.

In a further embodiment, the present invention discloses a method of manufacturing a MN system 100 which comprises:
- fabrication of the flexible and conductive anodic and cathodic MN patches, 3 respectively 40 provided with the Ag and AgCl electrodes respectively, as described above, where appropriate 3D printing methods will be described for the deposition of the carbon reinforced polymeric substrate and MN array of both MN patches, as well as for the deposition of the Ag/AgCl electrodes of the two patches, as described above;
- fabrication and direct deposition on the needles-containing surface of the MN patch 3 of an active nanofibrous reservoir defined as a crosslinked, biocompatible and biodegradable electrospun nanofibrous layer consisting of polymeric matrix and positively charged nanoparticles encapsulating therapeutic agent.
- on skin-planarized assembly of the tandem MN patch 3 - active nanofibrous reservoir 6 for a reliable fabrication and operation of the MN system 100 which should be ready to be electrically connected to the voltage source for injecting a safe dc electric current of about 0.5 mA to the skin.
- protection of the anodic 3 and cathodic 40 MN patches of the MN system 100 with a kinesiology tape 21 for preserving the fix position of the needles inside the epidermal layer 9 during iontophoresis-enhanced transdermal delivery.

### 4. BRIEF DESCRIPTION OF DRAWINGS

Understanding of the present invention will be facilitated by the detailed reference to the accompanying drawings, which are incorporated herein and form a part of this specification. The drawings illustrate one or more embodiments of the invention and, together with the detailed description and the claims, serve to explain the principles and features of the invention, as follows.
Fig. 1. Cross-section view (A) of the iontophoresis-enhanced MN system 100 and its electric circuit containing the anodic MN patch embedding the therapeutic agent reservoir and the cathodic MN patch (no reservoir attached to it) and Exploded view (B) of the flexible, conductive non-dissolvable anodic MN patch of an iontophoresis-enhanced MN-based system, with the active nanofibrous reservoir layer separately processed and transferred subsequently directly to the skin, as per this invention.

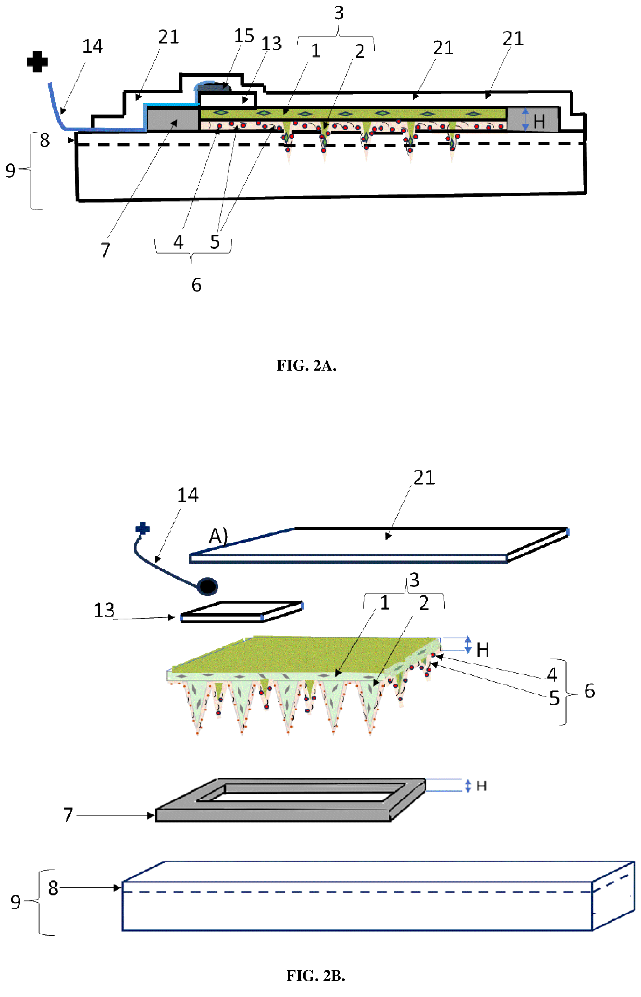

Fig. 2. Cross-section view (A) and Exploded-view (B) of the flexible, conductive non-dissolvable anodic MN array for iontophoresis-enhanced transdermal therapeutic agent delivery, with active nanofibrous reservoir layer deposited directly on the microneedles-containing surface of the anodic MN patch, as per this invention.
Fig. 3. (A) Perspective, magnified view of the flexible, conductive, non-dissolvable anodic MN patch for iontophoresis-enhanced transdermal therapeutic agent delivery, with active nanofibrous reservoir layer deposited directly on the anodic MN patch, and (B) The schematic presentation of the formulation and electrodeposition and crosslinking of the active nanofibrous reservoir layer directly on the anodic MN patch.
Table 1. Synthesis parameters of the carbon-reinforced polymeric inks for the 3D printing of the substrate and microneedle array of the flexible, conductive anodic and cathodic MN patches.

### 3. DETAILED DESCRIPTION

The following detailed description refers to the accompanying drawings, which illustrate specific embodiments and aspects in which the present invention may be implemented. These embodiments are described in sufficient detail to enable a person skilled in the art to make and use the invention. However, the invention is not limited to the disclosed embodiments and exemplary materials described for supporting the innovative concept. Various modifications and alternative configurations, including even other similar flexible materials, may be adopted without departing from the scope of the invention as defined by the appended claims. Furthermore, the embodiments described herein are not necessarily mutually exclusive; features from one embodiment may be combined with features of one or more other embodiments to form additional embodiments and remain within the scope of the invention. In addition, for a clear understanding of the invention a set of definitions is presented below, as follows.

### Definitions

As used herein, the term "epidermis" refers to the outermost layer of the skin forming a protective barrier and comprising the stratum corneum and viable epidermal layers. In the present invention, penetration into the epidermis denotes microneedles or agent delivery through one or more of these layers without reaching the dermis.

As used herein, the term "therapeutic agent" refers to any compound or molecule administered to prevent, treat, manage, or alleviate the symptoms or progression of a disease or medical condition. This includes agents intending to cure a disease, mitigate its effects, or enhance the quality of life of a subject in need thereof. Therapeutic agents may include, but are not limited to, small molecules/drugs, peptides, proteins, genes, or nucleic acids (such as DNA, RNA, siRNA, or mRNA), and may be hydrophilic or lipophilic in nature, depending on the intended application. Within this patent, the therapeutic agent is embedded in the positively charged nanoparticle to be defined below.

As used herein, the term "polycationic polymers" refers to polymers that bear multiple positive charges along their molecular backbone or side chains, typically arising from primary, secondary, tertiary, or quaternary amine groups. Such polymers may be biocompatible and may be either biodegradable or non-biodegradable, depending on the application. Exemplary polycationic polymers include, but are not limited to, linear or branched polyethyleneimine (PEI), chitosan and its derivatives, poly (β-amino esters), cationic dendrimers, polycationic peptides, and other natural or synthetic macromolecules possessing multiple cationic functionalities. These polymers are able of complexing nucleic acids via electrostatic interactions to form stable polyplexes and can also encapsulate other therapeutic agents that may not carry negative charges through self-assembly or assembly processes such as nanoprecipitation, emulsion-solvent evaporation, or other nanofabrication techniques suitable for generating stable nanoparticles.

As used herein, the term "cationic lipids" refers to amphiphilic compounds comprising at least one hydrophobic moiety and at least one cationic headgroup, wherein the cationic headgroup may be a quaternary ammonium, tertiary amine, guanidinium, or other positively charged group. These molecules are capable of self-assembling or assembling into liposomal or nanoparticulate structures and of electrostatically interacting with negatively charged biomolecules, including nucleic acids, to form lipoplexes or lipid nanoparticles (LNPs). Representative cationic lipids include but are not limited to, N-[1-(2,3-dioleyloxy)propyl]-N,N,N-trimethylammonium chloride (DOTMA), 1,2-dioleoyl-3-trimethylammonium-propane (DOTAP), 3β-[N-(N',N'-dimethylaminoethane)-carbamoyl] cholesterol (DC-Chol), 1,2-dioleoyl-3-dimethylammonium-propane (DODAP), 1,2-dioleyloxy-3-dimethylaminopropane (DODMA), dioctadecylamidoglycyl spermine (DOGS, Transfectam^{®}) and other analogues.

As used herein, in agreement with what was said above, the term "positively charged nanoparticle" refers to any particle having a size in the nanometric range, typically between 10 nm and 100 nm, possessing sufficient stability and a positive electrokinetic (zeta) potential in colloidal dispersions, and which is composed of the above defined biocompatible and polycationic polymers or cationic lipids to which the therapeutic agent is encapsulated/loaded, and which may be either hydrophilic or hydrophobic in nature.

Polymers are generally macromolecules composed of numerous repeating structural units, known as monomers, which are covalently bonded to form long molecular chains, that optionally contain pendant side groups. They can occur naturally or be synthetically manufactured, and they have a wide range of properties depending on their structure and composition.

Percolation threshold is the minimum concentration of a filler incorporated into a material which is dramatically changing its properties. Specifically, within this patent, the percolation represents the minimum concentration of a conducting nanoparticle (filler) incorporated in a non-conducting (dielectric) material, at which its electrical conductivity goes from non-conducting to the conducting state, i.e., its electrical conductivity increases by at least 6-8 orders of magnitudes, due to a huge number of conductive paths created between two electrodes by the interconnected fillers.

According to the brief description of the drawings, Fig. 1 A) and its reference numerals associated to each constructive element show a diagram of an iontophoresis-enhanced MN-based system 100, based on anodic MN patch containing the active nanofibrous reservoir and cathodic MN patch, which is used for transdermal delivery of therapeutic agents, and Fig. 1 B) shows an exploded view of the anodic MN patch from Fig. 1 A), as per one embodiment of this invention. A brief description of each component of the disclosed MN system 100 from Fig. 1 A) is presented below, as follows. This system 100 comprises the flexible, electrically conductive anodic MN patch 3, which consists of the substrate 1 and the array of the microneedles 2, where both the substrate 1 and the microneedles 2 are made of the same flexible, electrically conductive, biocompatible, and water insoluble material, by means of different technological processes like 3D printing method or micromolding, and the entire fabrication process and the design aspects of the microneedles will be described in detail, later. The anodic MN patch 3 of the MN system 100 comprises also the active nanofibrous reservoir 6, shaped as an electrospun nanofibrous layer. The active nanofibrous reservoir 6 is configured to incorporate in its matrix the positively charged, biocompatible nanoparticles 4 encapsulating the therapeutic agents such as drugs or nucleic acids, which are embedded in or grafted on the surface of the polymeric nanofibers 5. The details of the therapeutic agent nano-formulation and of the entire fabrication process of the active nanofibrous reservoir 6 will be described in the subsequent paragraphs. The distinctive feature of the embodiment described by Fig 1 A) and B) comes from the fact that the active nanofibrous reservoir 6 is processed separately from the MN patch 3 and then it is transferred and applied directly to the skin. From this Fig. 1 A), one can also see that the active nanofibrous reservoir 6 is surrounded by a dielectric adhesive and sealant layer 7, which will avoid any leak of therapeutic agent of the active nanofibrous reservoir 6 outside the anodic MN patch 3, during MN system 100 operation. As will be shown later, the embodiment described by MN system 100, from Fig.1 A) corresponds to the case when the active nanofibrous reservoir 6, shaped as a nanofibrous layer is prepared separately and then transferred to the skin. Here, for simplification, the skin is represented only by (i) stratum corneum layer 8, with its thickness of 10-30 µm, which is in contact with the environment, which layer is superposed on (ii) the other layers (lucidum, granulosum, spinosum, basal) of epidermis 9, , which have in total a thickness of 30-2000 µm, depending on its location on the body. After the active nanofibrous reservoir 6 is placed on the stratum corneum surface 8 of epidermis 9, the entirely processed anodic MN patch 3 (with dielectric adhesive layer 7 and electrodes elements 13, 14, 15 already attached to the MN patch 3) is applied on the stratum corneum 8, by surrounding and sealing tightly the active nanofibrous reservoir 6. As per Fig. 1 A). For the completion of the description of the iontophoresis-enhanced MN system 100 from Fig. 1 A), the electric circuit connecting the MN system 100 and the voltage supply 10, consisting of the anode 11 (the positive terminal) and the cathode 12, (the negative terminal) will be shown, as follows. A silver electrode layer 13 is deposited on the edge of the electrically conductive substrate 1, on the back side of the MN patch 3. A fine conductive wire 14 is bonded to Ag layer 13 and sealed with conductive silver epoxy 15. This wire 14 will electrically connect the anodic MN patch 3 to the anode 11 of the power supply 10. Thus, an excellent, conductive path is assured from the voltage source to the tip of each microneedle, for an efficient iontophoresis process. To close the electric circuit between the voltage source through the skin, an electrically conductive and flexible cathodic MN patch 40 consisting of substrate 17 and microneedles 2 (no fibrous reservoir) which is attached to surface of stratum corneum 8, via an electric current conducting epoxy layer 22 is also included. The cathodic MN patch 40 is prepared separately by the same technological method as for anodic MN patch 3. Thus, the AgCl electrode layer 18 is deposited on the upper surface of cathodic MN patch 40 and subsequently connected to the cathode terminal 12 via the conducting wire 20, which is sealed by Ag conductive epoxy adhesive 19 for a good mechanic and electric contact between wire 20 and the cathodic MN patch 40. Thus, the electrical circuit is closed via the epidermis 9, and this circuit will allow the positively charged nanoparticles loaded with therapeutic agent to be directed to the epidermis 9 via electrostatic repulsion performed by the positive charged anode 11, while the electrons 16 (generated by the oxidation reactions of the Ag atoms at the Ag electrode 13) to flow through wires 14 and 20 from the anodic MN patch 3 to the conductive cathodic MN patch 40 for the reduction of AgCl electrode 18. Finally, a very important component for safe operation of the iontophoresis enhanced MN system 100 is the kinesiology tape 21, which is applied above both the anodic MN patch 3 and cathodic MN patch 40 for preserving a fix position of all the microneedles 2 of both patches in the epidermis 9, during therapeutic agent delivery via iontophoresis operation. It is very important to mention here, that by using a cathodic MN patch 40 provided with microneedles 2 which will penetrate the stratum corneum 8, it will have a major impact on reducing the electrical resistance associated to the entire iontophoresis circuit of the MN system 100, by shunting the high electrical resistance of the stratum corneum 8. Thus, a lower voltage value of the power supply 10 will be necessary for obtaining a 0.5 mA current usually applied in the circuit for iontophoresis process in transdermal drug delivery.
Fig. 1 B) shows a perspective exploded view of the iontophoresis enhanced MN patch 3 pointing out very clearly all its component parts, which were described in more detail in Fig. 1 A). The reference numerals in Fig.1 B are identical to those from Fig. 1 A, so that the correspondence between the two figures can be done quite easily. Fig 1 B) is self-explanatory, but here one can easily understand that the entire active nanofibrous reservoir 6 is fully surrounded by the dielectric adhesive 7, so that no leak outside the MN patch 3 may occur. Such a dielectric adhesive layer 7 is very important for directing the entire current through the microneedle area only, and thus to better control the electric current distribution required by iontophoresis. In addition, from this Fig. 1B, one can better understand that the thickness "H" of the active nanofibrous reservoir layer 6 should be approximately equal to the thickness "H" of the dielectric adhesive 7 used for fixing and sealing the anodic MN patch 3 on the stratum corneum 8, and thus providing a planarization of the anodic MN patch surface 3 on the skin. In case of a higher thickness of the active nanofibrous reservoir layer 6 with respect to the thickness of the adhesive layer 7, a central dome may appear on the anodic MN patch 3, and this may prevent penetration of the microneedles 2 at the right deepness beyond the stratum corneum 8 of epidermis 9 and compromise the entire process. The design of the height of the microneedles will be done so that the microneedles penetrate the reservoir layer 6 and stratum corneum 8, and then stop in dermis 9, so that needles do not reach the nerve endings and blood vessels from hypodermis (not shown here). This explanation emphasizes once more the importance of kinesiology tape 21 in keeping tight the anodic and cathodic MN patches 3 and 40 respectively, inside the epidermis 9, during the entire iontophoresis process.

Fig. 2 discloses a cross-section view (A) and an exploded view (B) of the flexible and conductive anodic MN array for iontophoresis-enhanced transdermal therapeutic agent delivery, with active nanofibrous reservoir layer 6 deposited directly on the surface containing the needles 2 of the MN patch 3, as per this invention. All the reference numerals from Fig. 1 are kept also in Fig. 2, but there are important differences between the fabrication and assembly process of the flexible conductive anodic MN patch 3 in this embodiment in terms of (i) direct electrospinning of the active nanofibrous reservoir layer 6 on the surface of the flexible conductive anodic MN patch 3 above the microneedles 2, as shown in Fig. 2B and described with more details in the next paragraphs, and (ii) of positioning the entire anodic MN patch 3 inside the surrounding rectangular perimeter defined by the dielectric adhesive layer 7, from Fig. 2. The planarization of the anodic MN patch 3 after sealing it on stratum corneum 8 is an essential requirement, again for the efficient operation of the iontophoresis-enhanced transdermal delivery of therapeutic agents. As shown clearly in Fig. 2A and Fig. 2B , the thickness of the dielectric adhesive 7 for sealing the therapeutic agents loaded into active nanofibrous reservoir 6 deposited directly on the surface of the MN patch 3 should be equal to the sum of the thickness of the base layer 1 of the anodic MN patch 3 and the thickness of the active nanofibrous reservoir layer 6, so that to assure penetration of the needles to the right position beyond the stratum corneum 8 into the epidermis 9, but not reaching the dermis layer, which is located below the epidermis, and where nerves endings are present, so that to avoid any pain to the patient.

Fig. 3 A shows a perspective, magnified view of the flexible, conductive, non-dissolvable anodic MN patch 3 for iontophoresis-enhanced transdermal delivery of therapeutic agents, with active nanofibrous reservoir layer 6 deposited directly on the needle-containing surface of the conductive anodic MN patch 3. Fig. 3 B) shows a simplified presentation of the formulation and electrodeposition of the active nanofibrous reservoir layer 6, which is directly deposited on the anodic MN patch 3. This electrospinning deposition of the active nanofibrous reservoir layer 6 on the anodic MN patch 3 is possible as the anodic MN patch 3 has the substrate layer 1 and needles 2 which are electrically conductive, and thus the back surface of the anodic MN patch 3 makes an ohmic contact to the negatively biased bottom electrode collector 29 of the electrospinning equipment. In Fig. 3B, this equipment is represented symbolically by a syringe 25 containing precursor system 27 of the future active nanofibrous reservoir layer 6, which consists of therapeutic agent loaded-positively charged nanoparticles 4, dispersed within the solution of methacrylated gelatin (GelMA)-based matrix 26. The syringe 25 is connected to a high positive potential (about 16 kV) with respect to a negatively biased (- 4 kV) bottom electrode collector 29 on which the conductive anodic MN patch 3 is placed during electrospinning deposition process. The stabilization of the active nanofibrous reservoir 6 is performed during the electrospinning process, by in situ photopolymerization, utilizing a UV light source 28, operating within a wavelength range of 365-465 nm and an appropriate intensity. The UV radiation is applied continuously throughout the electrospinning procedure and is directed toward both the needle tip 30 of the syringe 25 and the full trajectory of the forming fibers up to the bottom electrode collector 29.
As shown in Fig. 3 B), and in agreement with the definitions from above, the biocompatible positively charged nanoparticles 4 encapsulating the therapeutic agents are obtained by assembling or self-assembling processes such as the electrostatic complexation of a polycation (e.g., polyethyleneimine-PEI) with a negatively charged molecule (e.g., drugs, nucleic acid - DNA, mRNA), and are loaded in or on the GelMA fibers 5 as shown in Figs. 1-3. During the electrospinning process these positively charged nanoparticles 4 are-dispersed in the precursor system 27. According to their chemical nature, these positively charged nanoparticles 4 serve to stabilize and protect the loaded therapeutic agents against premature enzymatic degradation and environmental inactivation under physiological conditions, as well as facilitating and improving its loading and release. Therefore, the specially designed architecture of the active nanofibrous reservoir 6, enables high-capacity loading and facilitates sustained, controllable transdermal delivery, thereby improving bioavailability and enhancing the pharmacological and therapeutic efficacy of the administered therapeutic agents. Furthermore, the iontophoresis-enhanced MN system 100 enables dual-controlled, on-demand release, through the synergistic mechanisms of (i) degradation of the active nanofibrous reservoir matrix 6 and passive diffusion through the epidermis 9 (as shown in Figs. 1-3) and (ii) externally applied iontophoresis, both processes allowing spatiotemporal regulation of the dose and enhanced release kinetics. The matrix of the active nanofibrous reservoir 6 gradually degrades at the contact with stratum corneum 8, allowing timed release of the therapeutic agents encapsulated in the positively charged nanoparticles 4, while the iontophoresis unit enables electrical current modulated delivery rate. MN system 100 is biocompatible, painless (with the inserted needles not touching the superior nerve terminations or the sensorial neuro-receptors from the dermis and below it (hypodermis), minimally invasive, and suitable for a broad range of therapeutic applications, including drug delivery, gene therapy, and localized treatment of skin diseases. More details about the fabrication technology of the flexible, conductive, non-dissolvable anodic MN patch 3 and active nanofibrous reservoir 6 will be now presented below.

For the practical implementation of the novel iontophoresis-enhanced MN system 100 for the transdermal delivery of therapeutic agents presented in Figs 1-3, dedicated methods will be described now for preparing both the anodic and cathodic MN patches 3 and 40, respectively, and the active nanofibrous reservoir 6, as follows.

Based on a detailed intellectual property analysis, from the best of our knowledge, we think that this is for the first time when the reinforced polymeric materials are used for preparing a biocompatible, non-dissolvable, flexible, electrically conductive MN patches 3, containing both the reinforced polymeric substrates 1 and 17 and the reinforced microneedle arrays 2, for rendering a high electrical conductivity along the entire electric current path of the iontophoresis-based MN system 100, starting from the anode 11 of voltage source 10 up to the tip of the needles 2 of the anodic MN patch 3, and closing the circuit through the epidermis 9, the cathodic MN patch 40 , its needles 2 and cathode 12 of the voltage source 10. Such a novel application of the reinforced polymeric materials for biomedical devices has a strong fundamental basis in the previous utilization of the reinforced polymeric materials in electromagnetic shielding, sensors and microelectrodes, to give just a few examples. In addition, there is a rich literature, and a well-established technology for the reinforcement of the dielectric polymeric materials with nanocarbonic fillers aiming to identify the percolation threshold (see the above definition of the percolation threshold) of the carbonic fillers incorporated in the dielectric polymeric material and the changes of the mechanical and thermal properties of the nanocomposite formed by reinforcement. By doping the polymeric material with nanocarbonic fillers at the percolation threshold and above one gets a dramatic increase of the electric conductivity by more than 8-10 orders of magnitudes, and an increase in the mechanical properties like mechanical strength, Young's modulus, of the new nanocomposite thus formed. It is important to understand that percolation threshold should be always considered carefully as its value may vary as a function of aspect ratio of the carbonic filler, and the nature of the polymeric material, and method of mixing the filler inside the melt or the precursors of a certain polymeric material. This means that percolation threshold found by others for a certain reinforced material outside the claimed range by the authors of this invention, does not go outside the scope of this invention. In the next paragraphs a few examples of reinforcing technologies for preparation of the biocompatible, non-dissolvable, conductive MN patches 3 and 40, made of polyimide (PI), polyamides (PA) and polydimethylsiloxane (PDMS), reinforced with carbonic nanomaterials like carbon nanotube (CNT), carbon black (CB), carbon nanohorn (CNH) will be shown. For those skilled in the art, it is obvious that the list of polymeric materials and reinforcing fillers can be easily extended without departing from the scope of the invention. Generically, the preparation of the flexible conductive MN patches 3 and 40 is performed in two steps. In the first step, the liquid phase of the reinforced polymeric material is prepared, and in the second step, the appropriate 3D printing technology will be used for a maskless preparation of the flexible, conductive MN patches 3 and 40. An alternative to 3D printing is micro-molding technology where the liquid phase of the reinforced polymeric composite (the ink) is filling the recessed regions of the mold (made of metal or PDMS, for example, as described in the above prior of this invention), which represents the negative version of the "future" MN patch 3 and 40 containing both the substrate 1 and 17, respectively, and the array of needles 2 on each MN patch. Extraction from the mold of the reinforced nanocomposite after in-situ polymerization inside the mold will provide flexible conductive MN patches 3 and 40.

Here is an example of preparation of MN patches 3 and 40 obtained by reinforcing the polyimide (PI) with carbon nanotube (CNT), above percolation threshold. PI is recognized as a chemically, mechanically and thermally robust polymeric material with insulating properties. This was the reason for selecting it in the past for many applications, and for further improving its performance by adding electrical conductivity properties, and for further enhancing its mechanical properties. As mentioned above, the first stage is the preparation of the CNT-PI ink, and the second step is the 3D printing of it. As per literature data, by reinforcing PI with CNT, a percolation threshold of CNT in PI was found to be 0.2-0.4 vol% (~0.3-0.5 wt%), as proven in the paper of Xiaowen Jiang, Yuezhen Bin and Masaru Matsuo, Electrical and Mechanical Properties of Polyimide-carbon nanotubes composites fabricated by in-situ polymerization, Polymer 46 (2005) 7418-7424. In general, for a reliable chemical design of the conductive nanocomposite, the concentration of the nanocarbonic material in the final solid nanocomposite should be 20-50% higher than the percolation threshold. This will assure both high electrical conductivity and high mechanical strength needed in our MN patches 3 and 40, for penetration of the stratum corneum 8, but at the same time will increase the viscosity of the ink. Thus, if ink-jet 3D printing cannot be used due to increased ink viscosity, then for higher-viscosity inks, Direct Ink Writing (DIW) 3D printing is employed. The key requirements of achieving the PI-CNT nanocomposites with a homogeneous composition and high electrical conductivity are assured by a rigorous preparation method focused on the uniform dispersion of the filler inside the entire body of the nanocomposite. The ink of the PI-CNT nanocomposite can be obtained by either dispersion of the CNT suspension in the commercial ready-made Polyamic acid (PAA) solution, or, one-by-one by adding monomeric precursors of the PAA (pyromellitic dianhydride-PMDA and oxydianiline-ODA) to the CNT suspension, a process which should occur in a humidity-and-oxygen-free ambient. In-situ polymerization of the above final mixture will occur after the ink printing/micromolding process. The detailed process for preparing 1 gram of solid PI-CNT composite with 1 wt% concentration of CNT (above percolation threshold of about 0.5 wt%) in the composite can be briefly described here, as follows.
Disperse the 0.01 grams CNT above percolation threshold (e.g. 1 wt%) in 8 grams of N, N-dimethylacetamide (DMAc) and 0.005 grams polyvinylpyrrolidone (PVP) in ultrasonic bath for about 1-2 hours till a loose network is formed (this is an important step for homogeneity of the future nanocomposite).
1. Add the above dispersed CNT network to 6.6 grams of PAA commercial solution (for example PAA solution in about 5.6 grams of DMAc), while working under anhydrous conditions, in N₂ dry atmosphere, at low temperatures (5-10 °C). The alternative is to add stoichiometrically the monomers of PDMA and ODA for generating by chemical reaction 0.66 grams of PAA solution, in the dispersed CNT in DMAc). Use rigorous mechanical stirring plus planetary mixer for about 1 hour.
2. Adjust ink rheology (e.g. by adding the same solvent used in synthesis, or evaporation treatment) for an appropriate 3D printing process. At a CNT concentration of 1 wt% in PI, and with an ink containing 8 g of the liquid phase, ink-jet printing can still be used. For higher concentrations of the CNT in PI, the direct ink writing (DIW) should be used, due to higher viscosity of the liquid phase.
3. Printing process of PAA-CNT composite at room temperature as the PAA is stable.
4. For getting a final PI-CNT nanocomposite of higher concentration, the DIW method should be used, where the nozzle diameter should be 100-400 µm and print speed of 0.5-5 mm/s.
5. Post-Printing thermal imidization.
This thermal process will convert printed PAA-CNT structure into PI-CNT:
In the selection of the above amounts in grams of the precursors for obtaining 1 gram of solid PI-CNT nanocomposite (with CNT above percolation threshold) the following considerations have been done: 0.01 grams of CNT (1 wt% of CNT) and an amount of about 15% of the PAA solution (6.63 grams) will form about 1 gram of solid CNT-PI composite, as the solvent will be obviously evaporated and most of PVP will be decomposed during thermal imidization treatments.

Here, another example of preparation of the conductive MN patch 3 is obtained by reinforcing the polyamide (PA), or nylon, with carbon nanotube (CNT), with CNT concentration above percolation threshold, and thus another excellent polymeric material for the same application is disclosed. Here, specifically, the PA6 nylon will be considered for reinforcement. The methodology will be similar with that presented for PI reinforcement, and it will be briefly described below. The percolation threshold of approximately 3 wt% for CNT nanofillers in PA6 has been demonstrated in the paper of Cristina Caamano, Brian Grady, and Daniel E. Resasco, *"Influence of nanotube characteristics on electrical and thermal properties of MWCNT*/*polyamide 6,6 composites prepared by melt mixing",* **CARBON 50** (**2012**) **3694** -**3707**. Starting from these findings, here it is shown how the reinforcing of PA6 with CNT for getting 1 gram of PA-CNT nanocomposite with 4% CNT inside can be done.
1. Disperse an amount of 40 mg of CNT (CNT can be stabilized in 20 mg of PVP) in 5 mL of formic acid and sonicate the suspension for about 30 minutes.
2. Prepare the PA solution by dissolving a pellet of PA6 weighing 0.960 g in 5.6 grams of formic acid, obtaining 6.56 g of ink and vigorously stir for 1-2 hours at room temperature till the entire PA6 is dissolved.
3. Slowly add the above CNT dispersion into PA6 solution under stirring and continue mixing for 2 hours at room temperature or mild heating below 40°C.
4. Adjust ink rheology of the above mixture of CNT-PA6, considering the increased percolation threshold for CNT-PA6 composite, which will also influence the flow and viscosity properties of the ink. This means adding solvent or evaporating solvent till the viscosity is in the range of (0.5-5) Pa*s, which is suitable for the direct ink writing.
5. Print the above ink by Direct Ink writing (DIW), layer by layer for obtaining the MN patch 3, based on its design transferred into software-program of the DIW equipment.
6. Dry the printed MN patch 3 under fume hood overnight or in an oven at 50-60 °C for 2-4 h.
7. Cure the dried MN patch 3 at 80°C for 30 minutes, to remove traces of formic acid.
8. Thermally treat the MN patches 3 and 40 at 120-150°C to improve the PA6 chain mobility and structure.
The selection of specific amount of 5.6 grams of the PA6 solution is because about 15% of the liquid phase of PA6 solution (about 6.6 grams) will be converted into about 960 mg of PA6. If 40 mg of CNT is added to the above solid content, one gets the mass of the solid composite of PA6-CNT of about 1 gram, as initially indicated.

Here, another example is shown for the preparation of the iontophoresis enhanced flexible, conductive MN patches 3 and 40 for transdermal delivery of therapeutic agents is disclosed based on reinforcing the commercial polydimethylsiloxane (PDMS) with carbonic fillers above percolation threshold. We shall follow the same methodology as presented above. The percolation threshold of CNT in PDMS is typically higher than 0.5 wt% depending on the type of CNT, aspect ratio and mixing process. For this synthesis, the recipe for 2 wt% of CNT in the CNT-PDMS composite is given, in agreement with the percolation threshold found in recent literature (Jian Du, Li Wang, Yanbin Shi, Feng Zhang, Shiheng Hu, Pengbo Liu, Anqing Li and Jun Chen, Optimized CNT-PDMS Flexible Composite for Attachable Health-Care Device, Sensors 2020, 20, 4523; doi:10.3390/s20164523). Below, a process flow for preparing 1 gram of solid CNT-PDMS composite at the end of the curing process is shown.
1. Disperse 20 mg of CNTs in 200-500 µL of toluene or IPA, add 2 mg of PVP dispersant, and sonicate for 30-60 minutes in ice bath, to avoid overheating.
2. Add 891 mg PDMS base (e.g. Sylgard 184) slowly to the CNT dispersion, mix thoroughly by planetary mixer or mechanical stirring for 2-5 minutes, and then degas for 10-15 minutes in vacuum desiccator to remove bubbles.
3. Add 89 mg of commercial PDMS curing agent (PDMS base: PDMS curing agent=10:1), then mix gently the composite, and degas if air bubbles are noticed.
4. Adjust rheology of the liquid phase composite by the addition of fume silica (if needed), such that the viscosity of the ink will be within the range of 1-2 Pa*s for DIW printing, wherein shear-thinning behavior further occurs at low shear rates, as described in the previous recipes. In case of micromolding process, slightly lower viscosity may be targeted, e.g., below 1 Pa*s.
5. Load the well-prepared ink into the syringe of the DIW and perform the printing by means of a syringe nozzle of 300-800 µm, as per pre-loaded software program which will define the geometry of the MN patches 3 and 40, as designed. Alternatively, the micromolding may be carried out using a PDMS negative mold, which will be filled with the dedicated ink described at the above step.
6. Cure the printed MN patches 3 and 40, thus obtained at 60-80°C (standard commercial PDMS protocol) for 2 hours, and then perform post-curing at 100-120°C for final thermal consolidation of the conductive MN patches 3 and 40.

For those skilled in the art, it becomes obvious that the same methodology can be applied to a broader range of biocompatible polymeric materials, (e.g. such as polyetheretherketone (PEEK) and polymethyl methacrylate (PMMA), and a larger number of carbonic fillers (carbon black-CB, carbon nanohorn-CNH, carbon nano onions-CNO, carbon fiber-CF), without departing from the scope of this invention. As already mentioned, in the literature there is a dispersion in the values of the percolation threshold even for the same polymeric material and the same carbonic material used, depending on the aspect ratio of the filler and mixing method used. Therefore, the identification of other than the mentioned here percolation threshold values does not mean departing from scope of this invention. In accordance with these statements, in Table 1, an example of synthesis parameters and deposition printing methods of the PI, PA6, PDMS, each of these polymeric materials being reinforced with CNT, CNH, CB above the percolation threshold. The data from Table 1 are slightly different from those presented in the text due to small changes in the amount of solvent used for these calculations.

The next paragraphs will enter the details of the fabrication methods of the active nanofibrous reservoir layer 6 containing therapeutic agent-loaded positively charged nanoparticle 4, as presented in Figs. 1-3, as follows.

The active nanofibrous reservoir layer 6 consists of therapeutic agent-loaded positively charged nanoparticles 4 which are embedded in or grafted on the surface of the GelMA nanofibers 5 (Fig. 3 A, B). The modification of gelatin with methacrylic functionalities is well-documented in scientific literature, having been pioneered by Van Den Bulcke et al., who first reported the synthesis and characterization of photocrosslinkable methacrylated gelatin (GelMA) hydrogels (Van Den Bulcke, An I., Bogdan Bogdanov, Nadine De Rooze, Etienne H. Schacht, Maria Cornelissen, and Hugo Berghmans. "Structural and rheological properties of methacrylamide modified gelatin hydrogels."Biomacromolecules"1, no. 1 (2000): 31-38; DOI: 10.1021/bm990017d). Moreover, a detailed step-by-step protocol for the preparation of GelMA, including control over the degree of functionalization and mechanical properties, is also presented by Loessner et al. (Loessner, Daniela, Christoph Meinert, Elke Kaemmerer, Laure C. Martine, Kan Yue, Peter A. Levett, Travis J. Klein, Ferry PW Melchels, Ali Khademhosseini, and Dietmar W. Hutmacher. "Functionalization, preparation and use of cell-laden gelatin methacryloyl-based hydrogels as modular tissue culture platforms. "Nature protocols"11, no. 4 (2016): 727-746; DOI: org/10.1038/nprot.2016.037).

The active nanofibrous reservoir layer 6 employs GelMA - methacrylated gelatin - as a matrix owing to its nature as a gelatin derivative with photocrosslinkable, biodegradable, and biocompatible properties, combined with good electro spinnability that enables the formation of uniform and stable fibrous structures (Xue, Yumeng, Han-Jun Kim, Junmin Lee, Yaowen Liu, Tyler Hoffman, Yi Chen, Xingwu Zhou et al. "Co-electrospun silk fibroin and gelatin methacryloyl sheet seeded with mesenchymal stem cells for tendon regeneration. "Small" 18, no. 21 (2022): 2107714; DOI: 10.1002/smll.202107714). In addition, GelMA offers high versatility for incorporating a wide variety of therapeutic agents, either directly or via encapsulation in nanocarriers, and provides tunable mechanical properties. Importantly, it also allows precise modulation and control of therapeutic agent releases through careful adjustment of the degree of methacrylation, and consequently the degree of crosslinking. As presented in Fig 3B, the stabilization of GelMA nanofibers 5, respectively of the active nanofibrous reservoir layer 6 containing therapeutic agent-loaded positively charged nanoparticles 4 is performed by *in situ* crosslinking, *via* photopolymerization of GelMA in the presence of a photo-initiator, using a UV light source 28, during the electrospinning and active nanofibrous reservoir layer 6 deposition on the negatively biased (- 4kV) electrode/collector 29 or on the surface of the anodic MN patch 3. This process results in a structurally stable, conformal nanofibrous layer with desirable mechanical and degradation properties for dual-controlled therapeutic agent delivery *via* the synergistic combination of the iontophoresis coupled to the anodic MP patch 3 and degradation of the active nanofibrous reservoir matrix 6.
Generally, the precursor system 27 for electrospinning consists of a GelMA solution 26 in which therapeutic agent-loaded positively charged nanoparticles 4 are dispersed. GelMA solution is obtained by dissolving lyophilized GelMA in phosphate-buffered saline (PBS, pH 7.4) to achieve a final concentration 35% w/v. Here, fish gelatine is preferred to synthetize GelMA, owing to its lower gelling temperature (~10-20 °C) compared to mammalian gelatin (~30 °C) and capability to remain liquid at room temperature. The mixture is stirred magnetically at 40°C for approximately 2 hours to ensure complete solubilization and homogeneity, resulting in a clear, viscous solution. Once the solution becomes homogeneous and transparent, the photo-initiator, LAP (lithium phenyl (2,4,6-trimethylbenzoyl) phosphinate) or Irgacure 2959 (2-hydroxy-4'-(2-hydroxyethoxy)-2-methylpropiophenone) is added at a concentration of 1.0 % w/w relative to the protein content; in the context of the present invention LAP is preferred. The mixture is then stirred for an additional 30 minutes under light-protected conditions to ensure complete dissolution of the photo-initiator.

The therapeutic agent-loaded positively charged nanoparticles 4 may be incorporated into or on the GelMA nanofibers 5 through dispersion in the GelMA solution 26 either in solid form, after lyophilization, or in hydrated form, immediately after synthesis. In the latter case, therapeutic agent-loaded positively charged nanoparticles 4 are first concentrated through conventional techniques such as centrifugation, filtration, or other suitable concentration methods. For optimal dispersion of therapeutic agent-loaded positively charged nanoparticles 4 into the precursor system 27, non-ionic surfactants might be employed. Preferred surfactants include those with a hydrophilic-lipophilic balance (HLB) ≥ 10, such as Tween 20, Tween 80, Pluronics, polyethylene glycol (PEG) or polyvinyl alcohol (PVA) (MW 31000-50000, MW 30000-70000 hydrolyzation degree 87%). It should be noted that the concentration of therapeutic agent-loaded ppositively charged nanoparticles 4 dispersed into the GelMA solution 26 must be optimized to ensure an enhanced therapeutic effect without compromising the structural integrity of the active nanofibrous reservoir 6 during electrospinning and deposition onto the surface of the microneedle (MN) anodic patch 3 or on the negatively biased electrode/collector 29.

The therapeutic agent-loaded positively charged nanoparticles 4 may take different forms, including: (i) polyplexes, obtained by complexing nucleic acids (e.g., DNA, plasmid DNA, mRNA, ssRNA) with biocompatible polycationic carriers; (ii) lipoplexes, obtained by assembling nucleic acids with biocompatible cationic lipid carriers and (iii) drug-loaded polycationic nanoparticles, obtained by assembling drugs (e.g., dexamethasone, diclofenac, salicylic acid, insulin) with polycationic carriers. Suitable polycationic materials include, but are not limited to, linear or branched polyethyleneimine (PEI), chitosan, poly (β-amino esters), cationic dendrimers, and polycationic peptides and, also cationic lipids. In the following sections, examples of formulation strategies for preparing biodegradable and biocompatible active nanofibrous reservoir layer 6, based on GelMA fibers embedding polyplexes, lipoplexes and drug-loaded polycationic carriers, will be described. Thus, the fabrication of the active nanofibrous reservoir layer 6 is a two-step process: (i) assembly of therapeutic agent-loaded positively charged nanoparticles 4 along with their dispersion in the GelMA solution 26 to generate the precursor system 27 and (ii) electrospinning of the active nanofibrous reservoir layer 6 on the surface of MP patch 3 or on the negatively-biased (-4 kV) electrode/collector, 29. The constructed iontophoresis-enhanced transdermal delivery MN system 100 can be applied for local gene therapy, including skin cancers, melanoma, chronic wound treatment and cutaneous vaccination.

Here, the electrospinning process is conducted at a temperature of 25°C and relative humidity of 40%, using an IME Technology electrospinning system equipped with temperature and humidity control. Alternatively, any equivalent electrospinning system that provides these environmental conditions may be used. A volume of the prepared precursor system 27 is loaded into a vertical syringe 25 for electrospinning provided with a needle having 0.6 mm in inner diameter through which the solution was pushed with the help of a syringe pump. The distance between the needle 30 and the static negatively biased electrode collector 29 is maintained between 10-19 cm, preferably 10 cm. The applied potential difference can be between 15-20 kV, preferably 16 kV. The conductive anodic MN patch 3 is placed on the static collector 29, where a negative potential is applied (-4 kV), while a positive potential (up to 16 kV) is applied at the upper needle tip 30 of the electrospinning system, forming a Taylor cone. The resulting active nanofibrous reservoir layer 6, with a thickness of about 100 µm and with the GelMA nanofibers 5 diameter ranging between 100 nm and 300 nm is thus deposited on the surface of MN patch 3 or on the surface of electrode collector 29. In the latter case, the fibrous layer 6, is then transferred on the stratum corneum 8 of epidermis 9. Due to its fibrous nature, such a reservoir layer 6 has a high surface area.

Photopolymerization of the active nanofibrous reservoir layer 6 is carried out *in situ* during the electrospinning process, utilizing a UV light source 28 operating within a wavelength range of 365-436 nm (405 nm for LAP and 365 nm for Irgacure 2959) and an appropriate intensity. UV radiation is applied continuously throughout the electrospinning procedure and is directed toward both the Taylor cone at needle tip 30 and the full trajectory of the forming nanofibers up to the electrode collector 29. This ensures effective crosslinking of the methacrylated gelatin. The resulting anodic MN patches 3, coated with an active nanofibrous reservoir layer 6, are subsequently collected and stored at ambient temperature under dry conditions.

Here, it is an example of preparing active nanofibrous reservoir layer 6 embedding polyplexes as positively charged nanoparticles 4 and deposited on the anodic MN patch 3 described above. Specifically, the standard double strained DNA (2000 bp, Mw =1320*10³ g/mol) and linear-polyethylene imide (l-PEI, MW = 10.000 g/mol) will be considered for the assembly of polyplexes. DNA can be condensed spontaneously into individual stable aggregates by PEI through electrostatic interactions between anionic phosphate groups (P) from DNA and cationic amino groups (N) presented in the polymeric structure. As the complexation process is predominantly governed by strong electrostatic interactions, the stability and hydrodynamic properties of PEI/DNA polyplexes are mainly dependent on the nitrogen-to-phosphate (N/P) ratio. This relationship has been thoroughly investigated by Dai et al., who demonstrated that at an N/P ratio of ≥ 6, stable polyplexes containing a single DNA molecule can be obtained (Dai, Zhuojun, and Chi Wu. "How does DNA complex with polyethylenimine with different chain lengths and topologies in their aqueous solution mixtures? "Macromolecules" 45, no. 10 (2012): 4346-4353; DOI: 10.1021/ma2027963). Starting from these findings, the detailed methodology of preparing 3.2 mL of precursor system 27 containing polyplexes can be briefly described here, as follows:
1. Prepare polyplex nanoparticles 4 at an N/P ratio of 6 by mixing 20 µL of DNA solution (0.05 mg/mL DNA in Tris buffer, pH 7.4) with 7.83 µL of 1-PEI solution (1.0 mg/mL, pH 7.4) in 172.17 µL of Tris buffer (pH 7.4). To ensure complexation, slowly add the PEI solution, diluted in the calculated volume of medium, into the DNA solution under vortex mixing for approximately 20 seconds. Incubate the mixture at room temperature for about 30 minutes to complete the complexation process. The amounts are calculated to obtain 200 µL of polyplex solution, typically containing 10 µg of DNA.
2. Prepare the GelMA solution 26 containing the photo-initiator, by dissolving 1.75 g of GelMA in 3.25 mL PBS (pH 7.4) and then adding 17.5 mg of LAP and maintaining the mixture at 35 - 40 °C under under light-protected conditions and magnetic stirring until a homogeneous and transparent yellow solution is obtained.
3. Add the 200 µL of freshy assembled polyplex nanoparticles 4, to 3 mL GelMA solution 26 and homogenise by gentile magnetic steering and/or low-power ultrasonication bath, under light-protected conditions, constantly monitoring and keeping the temperature at 35 - 40 °C, avoiding overheating, in order to obtain the precursor system 27 containing the polyplex nanoparticles 4 and GelMA solution 26, as shown in Fig. 3B
4. Clean and dry the surface of the collector electrode 29 and place the MN patch 3 with the needles 2 oriented toward upper side, i.e., to the syringe 25. For the alternative case of fibrous layer 6 deposited directly on the collector electrode 29, just be assured that the collector electrode 29 is clean and dry.
5. Load the precursor system 27 in the syringe 25 of the electrospinning equipment and connect all the accessories required for electrospinning process (connect the syringe to pump, connect the needle 30, collector 29, and UV source 28).
6. Start the electrospinning process after adjusting the technical (feed rate, electrical potential, distance between the needle and collector, etc.) and ambient parameters (temperature and humidity) according to the viscosity and conductivity of the precursor system 27 and simultaneously initiate the crosslinking of generated electrospun GelMA nanofibers 5 embedding polyplex nanoparticles 4 by positioning and switching on the UV light source 28.
7. Stop the electrospinning equipment once the targeted volume is deposited and the required thickness of the active nanofibrous reservoir layer 6 is achieved. Subsequently, continue exposing the active nanofibrous reservoir layer 6 to the UV light source 28 for an additional 30 minutes to complete the crosslinking step.
8. Collect the anodic MN patch 3 with active nanofibrous reservoir layer 6, containing the polyplex nanoparticles 4 embedded in or on the nanofibers 5, if the electrospinning of the active nanofibrous reservoir layer 6 was accomplished on the surface of anodic MN patch 3 and dry it in an oven, if necessary.
9. Alternatively, collect the nanofibrous reservoir layer 6 from the collector electrode 29 and transfer it to the surface of stratum corneum 8 of the epidermis 9.
The volume of solution of polyplexes added 200 µL in the 3 mL GelMA solution 26 is calculated to yield a final DNA concentration of 3.125 µg/mL in the electrospinning precursor system 27; this concentration is not expected to alter precursor spinnability.

Here, another example of preparing active nanofibrous reservoir layer 6 embedding lipoplexes as positively charged nanoparticles 4 and deposited on the MN patch 3 described above. These lipoplexes may incorporate cationic or ionizable lipids, including but not limited to DOTAP (1,2-dioleoyl-3-trimethylammonium-propane), DOTMA (N-[1-(2,3-dioleyloxy)propyl]-N,N,N-trimethylammonium chloride), and DODAP (1,2-dioleoyl-3-dimethylammonium-propane), which facilitate nucleic acid condensation, cellular uptake, and endosomal escape, enabling efficient gene transfection and controlled intracellular release. Here, specifically, the anionic compound siRNA (silencing RNA) and DOTAP cationic lipid with a quaternary ammonium group, will be used to formulate the lipoplexes. Like polyplexes, the stability and hydrodynamic characteristics of the lipoplexes are driven by the N/P ratio, which has been reported to be ≥5 by Kanamori et al (Kanamori, Miyu, Kouji Hara, Eriko Yamazoe, Takaaki Ito, and Kohei Tahara. "Development of Polyvinyl Alcohol (PVA) Nanofibers Containing Cationic Lipid/siRNA Complexes via Electrospinning: The Impact of PVA Characterization." Nanomaterials" 14, no. 13 (2024): 1083; DOI: org/10.3390/nano14131083). The methodology will be similar with that presented above for the construction of active nanofibrous reservoir layer 6 loaded with polyplex nanoparticles, and it will be briefly described below:
1. Prepare lipoplex nanoparticles 4 at an N/P ratio of 5 by mixing siRNA solution with a DOTAP suspension in Milli-Q water, to obtain 200 µL of lipoplex solution containing 10 µg siRNA.
2. Warm the GelMA solution 26 containing the photo-initiator to 35-40 °C, the methodology of obtaining GelMA solution is like the above-described methodology for the active nanofibrous reservoir layer 6 embedding polyplex nanoparticles.
3. Pre-equilibrate the lipoplex stock to the same temperature as GelMA solution 26 to avoid osmotic shock, respectively lipoplexes deterioration.
4. Add the lipoplex nanoparticles into a small aliquot of GelMA and mix gently by pipetting up and down 10-20 times. Then, transfer this fraction into the bulk GelMA and homogenize by gentle pipetting or slow magnetic stirring under light-protected conditions, for obtaining the precursor system 27, containing this time lipoplexes as positively charged nanoparticles 4 and GelMA 6. Vortexing should be avoided to prevent lipoplex disintegration.
5. Clean and dry the surface of the collector electrode 29 and place the MN patch 3 with the needles 2 oriented toward the upper side, i.e., to the syringe 25. For the alternative case of fibrous layer 6 deposited directly on the collector electrode 29, just be assured that the collector electrode 29 is clean and dry.
6. Load the precursor system 27 in the syringe 25 of the electrospinning equipment and connect all the accessories required for electrospinning process (connect the syringe to pump, connect the needle 30, collector 29, UV light source 28).
7. Start the electrospinning process after adjusting the technical (feed rate, electrical potential, distance between the needle and collector, etc.) and ambient parameters (temperature and humidity) according to the viscosity and conductivity of the precursor system 27 and simultaneously initiate the crosslinking of generated electrospun GelMA nanofibers 5 embedding lipoplexes by positioning and switching on the UV light source 28.
8. Stop the electrospinning equipment once the targeted volume is deposited and the required thickness of the active nanofibrous reservoir layer 6 is achieved. Subsequently, continue exposing the active nanofibrous reservoir layer 6 to the UV light source 28 for an additional 30 minutes to complete the crosslinking step.
9. Collect the MN patch 3, now containing the active nanofibrous reservoir layer 6 embedding lipoplex nanoparticles 4 on or in nanofibers 5, if the electrospinning of active nanofibrous reservoir layer 6 was accomplished on the surface of anodic MN patch 3 and dry it in an oven, if necessary.
10. Alternatively, collect the nanofibrous reservoir layer 6 from the collector electrode 29 and transfer it to the surface of stratum corneum 8 of the epidermis 9
The selected amounts are like those of obtaining active nanofibrous reservoir layer 6 embedding polyplexes, to not alter the spinnability of the precursor system 27.

Here, another example of preparing electrospun reservoir film 6 embedding drug-loaded polycationic nanoparticles as positively charged nanoparticles 4 and deposited on the anodic MN patch 3 described above. Generally, drug-loaded polycationic nanoparticles may be obtained through assembly or self-assembly processes governed by intra- and intermolecular forces, including ionic interactions, dipole-dipole interactions, hydrophobic interactions, hydrogen bonding, van der Waals forces, or other non-covalent and supramolecular interactions. Suitable methods for the preparation of such nanoparticles include, but are not limited to, nanoprecipitation, single- or double-emulsion techniques followed by solvent evaporation, dialysis, ionic complexation, and ionic gelation. Here, curcumin (CM) a plant-derived polyphenol with strong biological activity, and the natural polycationic biopolymer chitosan (CS, MW, 100-150kDa; degree of deacetylation, 80%) will be specifically considered for the assembly of drug-loaded polycationic nanoparticles (CM/CS NPs) 4. The protocol for the formulation of CM/CS NPs with suitable hydrodynamic characteristics and encapsulation efficiency is detailed presented in the work of Zahiri et al. (Zahiri, Maria, Mehdi Khanmohammadi, Arash Goodarzi, Shima Ababzadeh, Morteza Sagharjoghi Farahani, Sanam Mohandesnezhad, Naghmeh Bahrami, Iraj Nabipour, and Jafar Ai. "Encapsulation of curcumin loaded chitosan nanoparticle within poly (ε-caprolactone) and gelatin fiber mat for wound healing and layered dermal reconstitution. "International Journal of Biological Macromolecules" 153 (2020): 1241-1250; DOI: org/10.1016/j.ijbiomac.2019.10.255). Starting from these findings, the methodology of preparing 3.2 mL of precursor system 27 containing drug-loaded polycationic nanoparticles 4 can be briefly described here, as follows:
1. Prepare CM/CS nanoparticles 4 by firstly dissolving chitosan in 1% acetic acid aqueous solution at a concentration of 1-2 mg/mL, under magnetic steering overnight for complete solubilization. Then, slowly add the CM solution (0.25 mg/mL in H₂O: C₂H₅OH, 70:30 v/v) to CS solution in equal volume ratio, under magnetic stirring to obtain homogenize mixture. Further, add an aqueous solution of sodium tripolyphosphate (TPP, 1 mg/mL) dropwise to the chitosan/curcumin mixture while stirring. The cationic chitosan interacts with anionic TPP, forming nanoparticles 4 encapsulating curcumin. Continue stirring for 30-60 minutes to allow complete nanoparticle 4 formation. Purify the CM/CS nanoparticles 4 by centrifugation to remove the supernatant containing unencapsulated CM, followed by washing with deionized water/buffer, and collected by lyophilization for storage.
2. Warm the GelMA solution 26 containing the photo-initiator to 35-40 °C, the methodology of obtaining GelMA solution is like the two above-described methodology for the active nanofibrous reservoir layer 6.
3. Rehydrate 0.3 mg of lyophilized CM/CS nanoparticles 4 in a small volume of PBS (prewarmed to 40 °C) and sonicate gently (bath sonicator, short pulses, total <2 min) to disperse aggregates; if using hydrated CM/CS nanoparticles 4, concentrate them to the target concentration using a centrifuge.
4. Gradually add the CM/CS nanoparticle suspension to the 3 mL of warm GelMA solution 26 dropwise while stirring slowly (300-600 rpm) to avoid introducing bubbles and under light-protected conditions, to obtain the precursor system 27. Alternatively, the GelMA solution 26 can be also slowly added to the CM/CS nanoparticle suspension, maintaining a temperature of 37-40 °C throughout to ensure that the GelMA remained fluid.
5. Allow the mixture to equilibrate 15-30 min under gentle stirring. If necessary, apply low-power probe sonication briefly (e.g., 5-10 s bursts) to break small aggregates and ensure homogeneous distribution of CM/CS nanoparticles 4 within the GelMA solution 26, generating the precursor system 27. Overheating should be avoided to prevent particles deterioration while light-protected conditions should be maintained.
6. Load the precursor system 27 in the syringe 25 of the electrospinning equipment, degas briefly if necessary, and connect all the accessories required for electrospinning process (connect the syringe to pump, connect the needle, collector, UV source).
7. Clean and dry the surface of the collector electrode 29 and place the MN patch 3 with the needles 2 oriented toward the upper side, i.e., to the syringe 25. For the alternative case of fibrous layer 6 deposited directly on the collector electrode 29, just be assured that the collector electrode 29 is clean and dry.
8. Start the electrospinning process after adjusting the technical (feed rate, electrical potential, distance between the needle and collector, etc.) and ambient parameters (temperature and humidity) according to the viscosity and conductivity of the precursor system 27 and simultaneously initiate the crosslinking of generated electrospun GelMA nanofibers 6 embedding CM/CS nanoparticles 4 by positioning and switching on the UV light source 28
9. Stop the electrospinning equipment once the targeted volume is deposited and the required thickness of the active nanofibrous reservoir layer 6 is achieved. Subsequently, continue exposing the active nanofibrous reservoir layer 6 to the UV light source 28 for an additional 30 minutes to complete the crosslinking step.
10. Collect the anodic MN patch 3, now containing the active nanofibrous reservoir layer 6 embedding CM/CS nanoparticles 4 on or in the nanofibers 5, if the electrospinning of active nanofibrous reservoir layer 6 was accomplished on the surface of anodic MN patch 3 and dry it in an oven, if necessary.
11. Alternatively, collect the nanofibrous reservoir layer 6 from the collector electrode 29 and transfer it to the surface of stratum corneum 8 of the epidermis 9
The amount of 0.3 mg CM/CS nanoparticles 4 dispersed in 3mL GelMA solution 26 in calculated to represent approximately 10% w/v of drug-loaded polycationic nanoparticles 4 in the precursor system 27, ensuring that the spinnability of the fibers is not adversely affected, according to the protocol presented by Zahiri (Zahiri, Maria, Mehdi Khanmohammadi, Arash Goodarzi, Shima Ababzadeh, Morteza Sagharjoghi Farahani, Sanam Mohandesnezhad, Naghmeh Bahrami, Iraj Nabipour, and Jafar Ai. "Encapsulation of curcumin loaded chitosan nanoparticle within poly (ε-caprolactone) and gelatin fiber mat for wound healing and layered dermal reconstitution. "International Journal of Biological Macromolecules" 153 (2020): 1241-1250; DOI: org/10.1016/j.ijbiomac.2019.10.255).

## Claims

1. A flexible, electrically conductive microneedle-based system 100 for iontophoresis-enhanced transdermal delivery of therapeutic agents, comprising:
• A carbon-filler reinforced, flexible and non-dissolvable biocompatible substrate 1, 17;
• A plurality of carbon-filler reinforced non-dissolvable and biocompatible microneedles 2 attached to the substrates 1 and 17 for forming together the anodic microneedle patch 3 and cathodic microneedle patch 40, which are configured for painless insertion of microneedles 2 into the human epidermis 9;
• A biodegradable and biocompatible active nanofibrous reservoir layer 6 consisting of polymeric nanofibers 5 containing therapeutic agent-loaded positively charged, biocompatible nanoparticles 4 embedded in or grafted on said nanofibers 5;
• A kinesiology tape 21 for sealing the substrates 1, 17 of the microneedles 2 on the epidermis 9
• A carbon-filler reinforced flexible cathodic patch 40, containing the same type of microneedles, but no reservoir layer, attached also to the epidermis 9, and located in the proximity of the anodic microneedle patch 3;
• A voltage source 10 for supplying the electric current 16 in the electric circuit of microneedle-based system 100, consisting of microneedle patches 3, 40, wirings, 14, 20 and epidermis 9.

2. The microneedle-based system 100 of Claim 1, wherein the microneedle anodic patch 3 and cathodic microneedles patch 40 comprises one or more crosslinked polymers selected from: polyimide (PI), polyamine (PA), or polydimethylsiloxane (PDMS), which polymers are reinforced with carbon-based filler materials including, carbon nanotubes (CNTs, or, carbon nanohorns (CNHs) or, carbon black, graphene or, reduced graphene oxide,), or combinations thereof, incorporated into the substrate 1, 17 matrix and microneedles 2 at a concentration higher than the percolation threshold of the filler in the polymeric matrix.

3. The microneedle-based system 100 from Claim 1, wherein the therapeutic agent-loaded positively charged nanoparticles 4 of the active nanofibrous reservoir layer 6 consist of polyplex nanoparticles, or lipoplex nanoparticles, or drug-loaded polycationic nanoparticles, embedded in or grafted on the GelMA fibers 5.

4. The microneedle-based system 100 from Claim 1, wherein anodic patch 3 and the cathodic patch 40 are made by 3D printing, or micromolding.

5. The microneedle-based system 100 from Claims 1 and 3, wherein the active nanofibrous reservoir layer 6 embedding the therapeutic agent-loaded positively charged nanoparticles 4 is made by electrospinning of the precursor system 27 either directly on the anodic microneedle patch 3 or on the negatively biased electrode collector 29 followed by the transfer of the active nanofibrous reservoir 6 to the epidermis 9.

6. The microneedle-based system 100 from Claims 1 and 5, wherein the sealing of the active nanofibrous reservoir layer 6 inside the microneedle anodic patch 3 is performed with a dielectric adhesive layer 7 having a certain thickness calculated to assure the planarization of the anodic microneedle patch 3 after the insertion of the microneedles 2 in the epidermis 9, either for case of active nanofibrous reservoir layer 6 deposited directly on the anodic patch 3, or on the collector 29 of the electrospinning of instrument.

7. The microneedle-based system 100 from Claim 1, wherein the cathodic microneedle patch 40 is attached to the skin by an electrically conductive adhesive layer 22 to facilitate the flow of electric current 16 through the iontophoresis circuit.

8. The microneedle-based system 100 according to any one of Claims 1, 3, 5, wherein the therapeutic agent loaded positively charged nanoparticles 4 are released in a controlled and sustained manner.

9. The microneedle-based system 100 according to Claims 8, wherein the controlled release of therapeutic agents-loaded positively charged nanoparticles 4 is governed by the dissolution of the biocompatible and biodegradable active nanofibrous reservoir 27 upon contact with physiological fluids.

10. The microneedle-based system 100 according to Claims 8, wherein the enhanced, dual-controlled release of therapeutic agents-loaded positively charged nanoparticles 4 is further guided by iontophoresis, through the use of a voltage source 10 with its positive terminal 11 connected to the MN patch 3 sealing the fibrous reservoir 6 containing the polycationic nanoparticles (cationic repulsion) and its negative terminal connected to the cathodic MN patch 40.

11. A method for the fabrication of an electrically conductive, flexible and non-dissolvable biocompatible microneedle-based system 100 consisting of:
• Mixing the liquid polymeric precursors of the flexible substrate 1 with the carbonic material filler having a concentration higher than the percolation threshold of that filler in the final solid state of the electrically conductive substrates 1 and the microneedles 2.
• Adjusting the rheological properties of the above carbon-reinforced liquid precursor of the substrates 1, 17 and the microneedles 2 so that to be compatible with the requirements of the 3D printing method or micromolding.
• 3D printing or micromolding of the carbon-reinforced liquid precursor according to the design parameters for geometry of the anodic microneedle patch 3 and cathodic microneedle patch 17.
• Thermal annealing of the printed or molded carbon-reinforced liquid precursors for solvent removal and formation of the solid-state microneedle patches 3 and 17.
• Dissolving a predetermined amount of a biocompatible and biodegradable polymer and the required amount of photo-initiator in phosphate-buffered saline (PBS) to form a homogeneous polymer solution 26.
• Formulating the therapeutic agent-loaded positively charged nanoparticles 4 through assembly or self-assembly processes.
• Thorough dispersing the therapeutic agent-loaded positively charged nanoparticles within the polymer solution 26 for getting the precursor system 27.
• Loading the precursor system 27 in the syringe 25 of the instrument and performed the electrospinning process simultaneously with UV photopolymerization of the precursor system 27 for getting the active nanofibrous reservoir layer 6, deposited either directly on the anodic microneedle patch 3, or on the negatively-biased collector electrode 29 of the instrument, followed by the transfer of the active nanofibrous reservoir 6 on the epidermis 9.
• 3D printing of the Ag and AgCl electrodes on the upper surfaces of patches 3 and 40 respectively, and of the dielectric 7 and conductive 22 adhesive layers, respectively on the same surface with the microneedle array.
• Tight fixing MN patches 3 and 40 on epidermis 9 by sticking the kinesiology tape 21 above the MN patches 3 and 40 for keeping the inserted needles 2 firmly in the epidermis 9.
• Connecting the anodic 3 and cathodic 40 microneedle patches to the anode 11 and cathode 12, respectively, of the voltage source and set the selected electric current for the iontophoresis process.

12. Method from Claim 11, wherein the substrates 1 and 17 and microneedles 2 are made of polymeric materials like polyimide (PI) or polyamide (PA), or polydimethylsiloxane (PDMS) reinforced with fillers from carbon family like carbon nanotube or carbon black, or carbon nanohorn, or reduced graphene oxide, at concentrations higher than percolation threshold of the carbonic filler in the polymer matrix.

13. Method from Claim 11, wherein the active nanofibrous reservoir layer 6 consists of GelMA fibers 5 embedding in or on the fibers chemically stable therapeutic agent-loaded positively charged nanoparticles 4 like polyplexes, lipoplexes, or drug-loaded polycationic nanoparticles.

14. Method from Claim 11, wherein after mounting the kinesiology tape 21 on the upper surfaces of patches 3 and 40, a planar surface of the device is obtained on epidermis 9 because of the design of the thickness of the adhesive and active nanofibrous reservoir layer 6.

## Amended claims

### Amended claims in accordance with Rule 137(2) EPC.

1. A flexible, electrically conductive microneedle-based system (100) for iontophoresis-enhanced transdermal delivery of therapeutic agents, comprising:
- an anodic microneedle (MN) patch (3) and a cathodic microneedle (MN) patch (40), each comprising a carbon-filler reinforced, flexible and non-dissolvable biocompatible substrate (1, 17) and a plurality of carbon-filler reinforced, non-dissolvable biocompatible microneedles (2) attached to the substrate (1, 17);
wherein said anodic microneedle MN patch (3) contains a biodegradable and biocompatible active nanofibrous reservoir layer (6) consisting of polymeric nanofibers (5) containing therapeutic agent-loaded positively charged biocompatible nanoparticles (4) embedded in or grafted on said nanofibers (5),
and said cathodic microneedle MN patch (40) does not contain a reservoir layer (6);
- a kinesiology tape (21) for sealing the substrate (1, 17) on an epidermis (9), and
- a voltage source (10) for supplying an electric current (16) to the microneedle-based system (100), the voltage source (10) having a positive terminal (11) connectable to the anodic microneedle MN patch (3) and a negative terminal (12) connectable to the cathodic patch (40).

2. The microneedle-based system (100) according to Claim 1, wherein the microneedle MN anodic patch (3) and cathodic microneedle MN patch (40) comprise one or more crosslinked polymers selected from: polyimide (PI), polyamine (PA), or polydimethylsiloxane (PDMS), which polymers are reinforced with carbon-based filler materials including, carbon nanotubes (CNTs, or carbon nanohorns (CNHs) or carbon black, graphene or reduced graphene oxide), or combinations thereof, incorporated into the substrate (1, 17) and microneedles (2) at a concentration higher than the percolation threshold of the carbon-based filler material.

3. The microneedle-based system (100) according to Claim 1, wherein the therapeutic agent-loaded positively charged nanoparticles (4) of the active nanofibrous reservoir layer (6) consist of polyplex nanoparticles, or lipoplex nanoparticles, or drug-loaded polycationic nanoparticles, embedded in or grafted on the GelMA fibers 5.

4. The microneedle-based system (100) according to Claim 1, wherein the anodic patch (3) and the cathodic patch (40) are made by 3D printing, or micromolding.

5. The microneedle-based system (100) according to Claims 1 and 3, wherein the active nanofibrous reservoir layer (6) embedding the therapeutic agent-loaded positively charged nanoparticles (4) is made by electrospinning of a precursor system (27) either directly on the anodic microneedle MN patch (3) or on a negatively biased electrode collector (29) followed by the transfer of the active nanofibrous reservoir (6) to the epidermis (9).

6. The microneedle-based system (100) according to Claims 1 and 5, wherein the sealing of the active nanofibrous reservoir layer (6) inside the microneedle MN anodic patch (3) is performed with a dielectric adhesive layer (7) having a thickness calculated to assure the planarization of the anodic microneedle MN patch (3) after the insertion of the microneedles (2) in the epidermis (9), either for the case when the active nanofibrous reservoir layer (6) is deposited directly on the anodic patch (3), or when it is deposited on the collector (29).

7. The microneedle-based system (100) according to Claim 1, wherein the cathodic microneedle MN patch (40) is attached to the epidermis (9) by an electrically conductive adhesive layer (22) to facilitate the flow of an electric current (16) through the iontophoresis circuit.

8. The microneedle-based system (100) according to any one of Claims 1, 3, 5, wherein the therapeutic agent loaded positively charged nanoparticles (4) are released in a controlled and sustained manner.

9. The microneedle-based system (100) according to Claim 8, wherein the controlled release of the therapeutic agents-loaded positively charged nanoparticles (4) is governed by the dissolution of the biocompatible and biodegradable active nanofibrous reservoir (27) upon contact with physiological fluids.

10. The microneedle-based system (100) according to Claim 8, wherein the enhanced, dual-controlled release of the therapeutic agents-loaded positively charged nanoparticles (4) is further guided by iontophoresis, through the use of the voltage source (10) with its positive terminal (11) connected to the MN patch (3) sealing the fibrous reservoir (6) containing the polycationic nanoparticles (cationic repulsion) and with its negative terminal (12) connected to the cathodic MN patch (40).

11. A method for the fabrication of an electrically conductive, flexible and non-dissolvable biocompatible microneedle-based system (100) according to any of claims 1 to 10, the method comprising the following steps:
• Mixing liquid polymeric precursors of a flexible substrate (1) with a carbonic material filler having a concentration higher than the percolation threshold of that filler in the final solid state of an electrically conductive substrate (1, 17) and microneedles (2);
• Adjusting rheological properties of the above carbon-reinforced liquid precursor of the substrate (1, 17) and the microneedles (2) so that to be compatible with requirements of a 3D printing method or micromolding;
• 3D printing or micromolding of the carbon-reinforced liquid precursor according to design parameters for geometry of an anodic microneedle MN patch (3) and cathodic microneedle MN patch (40);
• Thermal annealing of the printed or molded carbon-reinforced liquid precursors for solvent removal and formation of solid-state microneedle MN patches (3) and (40);
• Dissolving a predetermined amount of a biocompatible and biodegradable polymer and a required amount of a photo-initiator in phosphate-buffered saline (PBS) to form a homogeneous polymer solution (26);
• Formulating therapeutic agent-loaded positively charged nanoparticles (4) through assembly or self-assembly processes;
• Thorough dispersing the therapeutic agent-loaded positively charged nanoparticles (4) within the polymer solution (26) for getting a precursor system (27).
• Loading the precursor system (27) in a syringe (25) and performing an electrospinning process simultaneously with UV photopolymerization of the precursor system (27) for getting an active nanofibrous reservoir layer (6), deposited either directly on the anodic microneedle MN patch (3), or on a negatively-biased collector electrode (29), followed by the transfer of the active nanofibrous reservoir (6) on an epidermis (9);
• 3D printing of Ag and AgCl electrodes on the upper surfaces of patches (3) and (40) respectively, and of dielectric (7) and conductive (22) adhesive layers, respectively, on the same surface of the patches (3) and (40) as the microneedles (2);
• Providing a kinesiology tape (21) for tight fixing said patches (3) and (40) on epidermis (9) by sticking above the MN patches (3) and (40) for keeping the inserted microneedles (2) firmly in the epidermis (9);
• Providing an iontophoresis unit comprising a voltage source (10) with a positive terminal (11) connectable to the anodic MN patch (40) and a negative terminal (12) connectable to the cathodic MN patch (3), wherein the iontophoresis unit is configured to control an electrical current through the epidermis and drive the enhanced delivery of the therapeutic agent-loaded nanoparticles (4) from the active nanofibrous reservoir (6) through the microneedle-created microchannels into the epidermis (9).

12. Method according to Claim 11, wherein the substrates (1, 17) and microneedles (2) are made of polymeric materials like polyimide (PI) or polyamide (PA), or polydimethylsiloxane (PDMS) reinforced with carbon-based filler materials like carbon nanotube or carbon black, or carbon nanohorn, or reduced graphene oxide, at concentrations higher than the percolation threshold of the carbon-based filler material .

13. Method according to Claim 11, wherein the active nanofibrous reservoir layer (6) consists of GelMA fibers (5) embedding, in or on said fibers (5), chemically stable therapeutic agent-loaded positively charged nanoparticles (4) like polyplexes, lipoplexes, or drug-loaded polycationic nanoparticles.

14. Method according to Claim 11, wherein after mounting the kinesiology tape (21) on the upper surfaces of patches (3) and (40), a planar surface of the device is obtained on epidermis (9) by the design of the thickness of the adhesive (7, 22) and of the active nanofibrous reservoir layer (6).

15. A method for transdermal delivery of one or more therapeutic agents using the transdermal delivery system (100) according to any one of claims 1 to 10, wherein the method consists of:
• applying and fixing with dielectric adhesive (7) on the surface of an epidermis (9) a conductive anodic MN patch (3) coated with the active nanofibrous reservoir (6), said reservoir (6) comprising positively charged nanoparticles (4) encapsulating said one or more therapeutic agents;
• applying and fixing with a conductive adhesive (22) a cathodic MN patch (40) provided with AgCl electrodes (18, 19, 20) to the epidermis (9), in the proximity of the anodic MN patch (3);
• connecting the electrodes of the anodic (3) and cathodic (40) MN patches to positive (11) and negative (12) terminals, respectively, of a voltage source (10) to establish an electrical circuit and allow a controllable electric current of about 0.5 mA to flow through said circuit containing the local dermal zone;
• activating an iontophoresis unit to facilitate safe dual-controlled release of the therapeutic agent, wherein such dual-controlled delivery is governed both by (i) electrical stimulation, and (ii) the gradual degradation of the biodegradable active nanofibrous reservoir (6).
